# EUROPÄISCHE PATENTSCHRIFT

(11) **EP 3 966 169 B1**
(45) Veröffentlichungstag und Bekanntmachung des Hinweises auf die Patenterteilung: **28.12.2022**
(21) Anmeldenummer: 20731790.0
(22) Anmeldetag: 11.05.2020
(51) Int. Cl.: C02F 1/32

(54) **LAMPENMODUL MIT LICHT EMITTIERENDEN DIODEN UND PHOTOREAKTOR**
LAMP MODULE COMPRISING LIGHT-EMITTING DIODES AND PHOTOREACTOR
MODULE DE LAMPE COMPRENANT DES DIODES LUMINESCENTES ET PHOTORÉACTEUR

(30) Priorität: 10.05.2019 DE 102019003299
(43) Veröffentlichungstag der Anmeldung: 16.03.2022
(73) Patentinhaber: Peschl Ultraviolet GmbH, 55130 Mainz (DE)
(72) Erfinder: PESCHL, Alexander, 55130 Mainz (DE); ZIEGENBALG, Dirk, 89081 Ulm (DE)
(74) Vertreter: mepat Patentanwälte
(86) Internationale Anmeldenummer: PCT/EP2020/000094
(87) Internationale Veröffentlichungsnummer: WO 2020/228980

(56) Entgegenhaltungen:
- DE-A1-102014 012 217
- DE-A1-102014 012 218
- DE-A1-102014 012 219

## Beschreibung

Die Erfindung betrifft ein Lampenmodul mit Licht emittierenden Dioden (LEDs), das als Tauchstrahler in Photoreaktoren zur Durchführung photochemischer Reaktionen oder auch in sogenannten AOPs ("advanced oxidation processes") sowie zu Desinfektionszwecken eingesetzt werden kann, sowie einen entsprechenden Photoreaktor.

Aus dem Stand der Technik ist bekannt, Strahlungsquellen, insbesondere auch UV-Strahler, als Tauchlampen in einem flüssigen Medium einzusetzen, um z. B. Wasser zu desinfizieren oder um photochemische Reaktionen durchzuführen. Herkömmlicher Weise werden in der Regel Niederdruck- oder Mitteldruckstrahler (Entladungslampen) eingesetzt, die z. B. zum Schutz vor Verschmutzung von einem Tauchrohr umhüllt werden, das für die emittierte bzw. für die photochemische Reaktion relevante Strahlung durchlässig ist. Das Emissionsspektrum der Entladungslampen kann durch geeignete Dotierung variiert und entsprechend der Anforderungen für die photochemische Reaktion in gewissem Umfang angepasst werden. Allerdings ist der Einsatz von Niederdruck- oder Mitteldruckstrahlern mit hohem Stromverbrauch verbunden und mit zunehmendem Alter lässt die Strahlungsintensität deutlich nach, ferner kann sich das Strahlungsspektrum verschieben, was eine regelmäßige Überprüfung in relativ kurzen Zeitabständen erforderlich macht.

Im Leuchtmittelbereich finden LEDs zunehmend Bedeutung aufgrund ihres vergleichsweise geringen Stromverbrauchs, hoher Lebensdauer und der hohen Schaltfestigkeit bei spontan vollem Lichtstrom. Die LED strahlt Licht, Infrarot- oder UV-Strahlung aus, wenn elektrischer Strom in Durchlassrichtung fließt. Die Wellenlänge der emittierten Strahlung hängt von der Dotierung des Halbleiterbauelements ab. Für UV-Strahlung kommen etwa Diamant, Aluminiumnitrid, Aluminiumgalliumnitrid oder Aluminiumgalliumindiumnitrid in Frage.

LEDs sind zwar keine Wärmestrahler wie etwa Mitteldruckstrahler, allerdings verkürzen hohe Temperaturen - gewöhnlich hervorgerufen durch hohe Ströme, die für eine maximale Lichtausbeute benötigt werden - die Lebensdauer der LEDs deutlich. Um diesen nachteiligen Einfluss der erhöhten Temperatur bei höheren Strömen zu vermeiden, werden LEDs häufig nicht bei Nennleistung, sondern darunter - verbunden mit geringerer Leuchtleistung - betrieben. Um dennoch eine gewünschte Lichtmenge zu erreichen, wird dann die Anzahl der eingesetzten LEDs erhöht.

Sollen die LEDs für eine hohe Lichtausbeute bzw. Strahlungsintensität mit hohen Strömen betrieben werden, ist eine effektive Wärmeableitung erforderlich, um die Lebensdauer der LEDs zu erhalten. Häufig werden Metallgehäuse, meistens aus Aluminium, zur Wärmeableitung eingesetzt.

Ein Leuchtmittel mit mindestens einer Lumineszenzdiode, die zur Wärmeabfuhr in einem in sich geschlossenen Gehäuse mit einer elektrisch nicht leitfähigen, transparenten Flüssigkeit umgeben ist, ist in DE 20 2010 014 116 U1 beschrieben. Die Flüssigkeit dient dem Wärmetransport von der Diode zu dem Gehäuse, von dem die Wärme großflächig abstrahlen kann. Mittels einer Pumpe kann die Flüssigkeit im Gehäuse umgewälzt werden, um die erwärmte Flüssigkeit von der Diode zur Gehäusewand zu transportieren, wo die Wärme in die Umgebung abstrahlen kann.

Für den Einsatz von LEDs in Photobioreaktoren zur Kultivierung von Mikroorganismen wird in der US 2010/0267125 A1 ein LED-Leuchtmodul beschrieben, bei dem die LEDs mittels eines Trägerrohrs gekühlt werden, das von einem transparenten Hüllrohr umgeben ist. In Bioreaktoren liegen im Reaktionsraum Druck- und Temperaturbedingungen vor, die sich nicht wesentlich von Umgebungsdruck und Raumtemperatur unterscheiden, da nur so üblicherweise ein optimales Wachstum der Mikroorganismen erreicht werden kann. Sollen LEDs in Photoreaktoren für chemische Synthesen, z. B. Photochlorierung oder Photobromierung zum Einsatz kommen, so muss ein entsprechendes Lampenmodul einer Tauchlampe den erhöhten Anforderungen hinsichtlich den umgebenden Reaktionsbedingungen genügen, die sehr deutlich von Umgebungsdruck und Raumtemperatur abweichen können.

Aus DE 10 2010 042 670 A1 ist ein doppelwandiges Tauchrohr zur thermischen Entkopplung einer Strahlungsquelle von dem umgebenden Reaktionsraum bekannt.

EP 3 183 493 B1, DE 10 2014 012217 A1, DE 10 2014 012218 A1 und DE 10 2014 012219 A1 offenbaren ein hinsichtlich der Handhabung des Wärmehaushalts verbessertes LED-Lampenmodul, das nicht nur unter Umgebungsbedingungen und im Labormaßstab, sondern als Tauchrohr zur photochemischen Synthese oder photokatalysierten Synthese, bei AOPs sowie zur Desinfektion, auch im industriellen Maßstab, eingesetzt werden kann. Dieses Lampenmodul weist einen Kühlkörper auf, an dessen Außenseite Trägerstrukturen mit LEDs angeordnet sind, und der einen innenliegenden Fluidpfad für einen Kühlmittelkreislauf aufweist, sowie eine Kammer für eine Stromversorgungs-/ Steuerungsleitung begrenzt. Der Kühlkörper mit den LEDs ist in einem doppelwandigen Tauchrohr aus einem für die Wellenlängen der von den LEDs des Lampenmoduls emittierten Strahlung durchlässigen Material angeordnet. Zur Verbesserung der thermischen Entkopplung mit dem doppelwandigen Tauchrohr kann der dazwischenliegende Spalt evakuiert oder mit einer Kühlflüssigkeit gespült werden. Ferner wird dort die Schaffung einer Inertgasatmosphäre in dem Tauchrohr mit einem gewissen Überdruck gegenüber dem Umgebungsdruck beschrieben, um den Explosionsschutz zu verbessern.

Ausgehend von diesem Stand der Technik ist es Aufgabe der vorliegenden Erfindung, ein weiter verbessertes Lampenmodul mit LEDs bereitzustellen.

Diese Aufgabe wird durch ein Lampenmodul mit den Merkmalen des Anspruchs 1 gelöst.

Weiterbildungen sind in den abhängigen Ansprüchen 2-14 ausgeführt.

Die weitere Aufgabe, einen Photoreaktor mit LEDs als Strahlungsquelle bereitzustellen, wird durch den Photoreaktor mit den Merkmalen des unabhängigen Anspruchs 14 gelöst.

Eine Ausführungsform eines erfindungsgemäßen Lampenmoduls, das dazu ausgebildet ist, als Tauchstrahler in photochemischen Reaktoren eingesetzt zu werden, weist einen Trägerkörper mit einer oder mehreren Licht emittierenden Diode (LED) auf. Die LED(s) kann/können direkt an dem Trägerkörper oder auf einer oder mehreren Trägerplatine(n), die an der Außenseite des Trägerkörpers angeordnet ist bzw. sind, befestigt sein. Ferner weist das Lampenmodul ein Kopfteil zum elektrischen Anschluss der zumindest einen LED und zur Halterung des Trägerkörpers auf, sowie ein Tauchrohr, das einen Tauchrohrinnenraum begrenzt, in dem der Trägerkörper mit der zumindest einen LED zum Schutz der LEDs vor den Reaktionsbedingungen im umgebenden Reaktionsraum angeordnet ist. Das Kopfteil weist ferner Anschlussleitungen für die Zufuhr und die Ableitung einer Kühlflüssigkeit auf, und der Trägerkörper ist als Kühlkörper ausgebildet, der zumindest einen internen Fluidpfad als Zufuhrabschnitt für die Kühlflüssigkeit begrenzt, der über das Kopfteil mit einer ersten Anschlussleitung verbunden ist. Erfindungsgemäß wird der von dem Tauchrohr begrenzte Tauchrohrinnenraum derart mit einer elektrisch nichtleitenden, d. h. elektrisch isolierenden Flüssigkeit gefüllt, dass die eine oder mehreren LED(s) vollständig in die elektrisch nichtleitende Flüssigkeit eingetaucht ist/sind. Dabei wird die elektrisch nichtleitende Flüssigkeit so ausgewählt, dass sie für zumindest eine Wellenlänge der von den LEDs des Lampenmoduls emittierten Strahlung transparent ist, die für die mit dem Lampenmodul durchzuführende Aufgabe vorgesehen ist.

Um eine optimale Kühlung der LEDs zu erreichen, indem nicht nur die von den LEDs erzeugte Wärme, sondern auch die Wärme infolge exothermer Reaktionen aus dem Reaktionsraum, der das Tauchrohr umgibt, mit der nichtleitenden Flüssigkeit abgeführt wird, kommunizieren die Kopfteil Anschlussleitungen für die Zufuhr und die Ableitung der nichtleitenden Flüssigkeit mit dem von dem Tauchrohr begrenzten Raum, indem der in dem Trägerkörper begrenzte Zufuhrabschnitt, der über das Kopfteil mit der ersten Anschlussleitung verbunden ist, an einer von dem Kopfteil abgewandten Seite des Trägerkörpers in den vom Tauchrohr begrenzten Tauchrohrinnenraum mündet und sich eine zweite Anschlussleitung durch das Kopfteil erstreckt und zum Tauchrohrinnenraum öffnet. Auf diese Weise kann die durch eine erste Anschlussleitung zugeführte nichtleitende Flüssigkeit bereits bei der Passage des Zufuhrabschnitts durch den Trägerkörper die von den LEDs erzeugte Wärme aufnehmen, strömt nach Austritt am Fußende des Trägerkörpers im Tauchrohr an der Oberfläche des Trägerkörpers mit den LEDs entlang nach oben, wobei die nichtleitende Flüssigkeit nicht nur weiter Wärme von den LEDs, sondern auch Wärme aus der Reaktorumgebung aufnehmen kann, bevor sie durch die zweite Anschlussleitung aus dem Tauchrohrraum abgeführt wird, um die aufgenommene Wärme außerhalb der Reaktorumgebung abzugeben.

Selbstverständlich kann mehr als ein Zulaufabschnitt durch den Trägerkörper vorgesehen sein, je nach Anzahl und Anordnung der LEDs kann beispielsweise pro axialer LED-Reihe ein Zulaufabschnitt durch den Trägerkörper vorgesehen sein.

So wird dieses Lampenmodul hinsichtlich der Kühlung der LEDs und der thermischen Abkopplung von der Umgebung verbessert und es stellt darüberhinaus eine erhöhte Gesamtlicht- bzw. Strahlungsleistung in Bezug auf die Lampen gemäß Stand der Technik bereit, da aufgrund der nichtleitenden Flüssigkeit die Photonen-Auskopplungseffizienz an der Phasengrenze Diodenoberfläche-Tauchrohrinnenraum vergrößert und die Reflexion an der Phasengrenze Tauchrohrinnenraum-Tauchrohrwand verringert wird. Dieser Effekt ist optisch bei Einsatz des Lampenmoduls dadurch feststellbar, dass das Tauchrohr für das Auge quasi unsichtbar wird.

Dadurch, dass die nichtleitende Flüssigkeit im Tauchrohrinneren permanent ausgetauscht wird, kann die von den LEDs und der Tauchrohrumgebung aufgenommene Wärme abgeführt und die Temperatur konstant gehalten werden. Besonders bevorzugt kann dabei eine Kreislaufführung der nichtleitenden Flüssigkeit vorgesehen werden, in dem die im Tauchrohr erwärmte nichtleitende Flüssigkeit im Kreislauf geführt wird, um außerhalb des Tauchrohrinnenraums die aufgenommene Wärme wieder abzugeben.

Dazu ist erfindungsgemäß vorgesehen, dass die die zu- und abführenden Anschlussleitungen zur Bildung des Kreislaufs miteinander verbunden sind, der ferner zumindest einen Wärmetauscher und eine Fördervorrichtung wie eine Pumpe und ggf. eine Absperrarmatur/Ventil aufweist.

In der vorliegenden Anmeldung wird die "elektrisch nichtleitende Flüssigkeit" kürzer als "nichtleitende Flüssigkeit" benannt, wobei aber immer die elektrische Nichtleitung gemeint ist. Wärme kann von, auf und durch diese nichtleitende Flüssigkeit übertragen werden. Als "transparent" werden hierbei alle Flüssigkeiten verstanden, die für die zumindest eine Wellenlänge der von den LEDs des Lampenmoduls emittierten Strahlung entlang der kürzesten Wegstrecke zwischen LED-Oberfläche und Tauchrohrinnenwand einen Transmissionsgrad von zumindest 75 % aufweisen.

Vorteilhaft wird durch das Eintauchen in die nichtleitende Flüssigkeit nicht nur eine Kühlung der LEDs bereitgestellt, die zur Verlängerung deren Lebensdauer beiträgt, sondern auf Grund des Brechungsindex' der nichtleitenden Flüssigkeit, der deutlich größer ist als der von Luft oder Inertgas und bei geeigneten nichtleitenden Flüssigkeiten im Bereich von etwa 1,35 bis etwa 1,55 (bei 20°C) liegt, sowohl die Photonen-Auskopplungseffizienz an den Phasengrenze Diodenoberfläche-Tauchrohrinnenraum vergrößert als auch die Reflexion an der Phasengrenze Tauchrohrinnenraum-Tauchrohrwand verringert werden, sodass die Gesamtlichtleistung des Lampenmoduls, d. h. die Strahlungsmenge und -dichte an der Außenfläche des Tauchrohrs deutlich erhöht ist.

Beim Befüllen des durch das Tauchrohr begrenzten Raums wird auch durch die nichtleitende Flüssigkeit eine explosionsfähige Atmosphäre vermieden. Vorteilhaft im Gegensatz zur Verwendung von einem Inertgas wie Stickstoff ist dabei, dass eine beschleunigte Alterung der Primäroptiken der LEDs vermieden wird, da gerade in Chemieanlagen VOCs ("volatile organic compounds") vorhanden sind, die in die üblicherweise als Silikonlinse ausgeführten Primäroptiken eindringen, diese eintrüben und somit die Lichtausbeute senken. Da die Primäroptiken erfindungsgemäß keiner gashaltigen Atmosphäre mehr ausgesetzt sind, sondern durch die nichtleitende Flüssigkeit abgeschirmt werden, wird der Alterungsprozess deutlich verlangsamt.

Die LED des Lampenmoduls wird entsprechend der für die durchzuführende Aufgabe vorgesehene(n) Wellenlänge(n) aus unterschiedlichen LEDs ausgewählt, die sich abhängig von ihrer Dotierung hinsichtlich der emittierten Strahlungswellenlängen unterscheiden und das sichtbare Licht- sowie Infrarot- und UV-Spektrum abdecken. Entsprechend wird die nichtleitende Flüssigkeit aus verschiedenen nichtleitenden Flüssigkeiten, die sich hinsichtlich der Absorptionsspektren unterscheiden, in Abhängigkeit der jeweilig eingesetzten Emissionswellenlänge gewählt, damit die nichtleitende Flüssigkeit transparent für die gewünschte Emissionswellenlänge ist.

Das erfindungsgemäße Lampenmodul bezieht sich auf LEDs als Lichtquelle. Hierbei sind alle Licht emittierenden Halbleiterbauelemente, auch organische Leuchtdioden (OLEDs), inbegriffen.

Das Tauchrohr ist sinnvollerweise aus einem für die Wellenlängen der von den LEDs emittierten Strahlung durchlässigen Material gefertigt. Zudem ist das Tauchrohr hinsichtlich Materialwahl und Formgebung auf Reaktionsdrücke im Bereich von Hochvakuum bis 6 bar Überdruck, die in dem das Tauchrohr umgebenden Reaktionsraum herrschen können, ausgelegt. Als Materialien kommen natürliches oder synthetisches Quarzglas bzw. Quarzglasmischungen oder Borsilikat, etc. in Frage.

Das Tauchrohr kann einseitig geschlossen sein, sodass das geschlossene Ende im Reaktionsraum des umgebenden Photoreaktors liegen kann, wie es häufig bei vertikaler Anordnung von Tauchlampen der Fall ist; das Tauchrohr kann aber auch beidseitig offen sein und auch horizontal in Reaktoren eingesetzt werden, wobei die offenen Enden des Tauchrohrs quasi aus dem Reaktionsraum ragen.

Um das Lampenmodul und die im Tauchrohr vorgelegte nichtleitende Flüssigkeit sicher von der Tauchrohrumgebung zu trennen, kann das Kopfteil in einer erfindungsgemäßen Ausführungsform mit dem offenen Ende eines einseitig geschlossenen Tauchrohrs, das vorwiegend in vertikaler Anordnung in einem Reaktor zum Einsatz kommt, abdichtend verbunden sein. Dazu können an zumindest einer Anlagefläche zwischen Kopfteil und Tauchrohr entsprechende Dichtmittel beispielsweise zumindest eine Dichtung angeordnet werden. Selbstverständlich kann ein Lampenmodul mit einseitig geschlossenem Tauchrohr, dessen offenes Ende mit dem Kopfteil abgedichtet verschlossen ist, auch in Orientierungen in einem Reaktor angeordnet werden, die von der Vertikalen abweichen. Wird ein beidseitig offenes Tauchrohr verwendet, so wird das eine Ende durch das abdichtende Kopfteil verschlossen und das andere Ende durch ein entsprechendes Verschlussteil.

Dabei kann die abdichtende Verbindung des Kopfteils mit dem Tauchrohr zusätzlich federnd gelagert sein, um Vibrationen zur Vermeidung von Glas-Metallkontakt zu dämpfen. Zusätzlich oder alternativ dazu kann eine formschlüssige Verbindung des Kopfteils mit dem Tauchrohr vorteilhaft sein, um Zugspannungen im Tauchrohrmaterial zu vermeiden. Beispielsweise kann das Kopfteil über Befestigungshilfsmittel wie beispielsweise Flansche, Ringe und Scheiben und federbelastete Verbindungsmittel an dem Tauchrohr befestigt werden.

In einer bevorzugten Ausführungsform kann das Tauchrohr mit dem Kopfteil an dem offenen Ende einen sich vom offenen Ende aus aufweitenden konusförmigen Kragen aufweisen, der von einem Haltering mit einer korrespondierend konusförmigen Öffnung an einem Basisring mittels federbelasteter Bolzen gehalten wird. Das Kopfteil ist in dem offenen Ende des Tauchrohrs aufgenommen und kann durch Schwenkriegel, die mit einer Ringnut an einer Innenwand des Tauchrohrs in Eingriff bringbar sind, oder bevorzugt durch einen Anpressring abdichtend befestigt werden, dessen Öffnung einen Durchmesser hat, der kleiner ist als der Durchmesser des Kopfteils und der auf dem Haltering angeordnet und zusammen mit dem Haltering an einem Basisring mittels federbelasteter Bolzen gehalten wird. Die Befestigung des Kopfteils im Tauchrohr ist erforderlich, damit das Kopfteil nicht durch den Druck, den die nichtleitende Flüssigkeit auf das Kopfteil ausübt, aus dem Tauchrohr gedrückt wird und die nichtleitende Flüssigkeit austreten kann. Auf diese Befestigung könnte verzichtet werden, wenn das offene Ende des Tauchrohrs von einem geschlossenen Gehäuse umgeben ist, das mit der nichtleitenden Flüssigkeit gefüllt werden kann, allerdings würde dadurch auch das Kopfteil in die nichtleitende Flüssigkeit eingetaucht, sodass das Austauschen oder Warten der LED-Lampe mit einem erhöhten Aufwand verbunden wäre.

Der konusförmige Kragen kann als separates Bauteil an dem offenen Ende des Tauchrohrs befestigt werden, oder das Tauchrohr kann vorzugsweise an dem offenen Ende mit dem konusförmig aufgeweiteten Kragen einstückig gefertigt sein. Die Befestigung mittels federbelasteter Bolzen verbessert die mechanische Stabilität des Lampenmoduls bei der abgedichteten Anordnung des Kopfteils und vermeidet Zugspannungen im Tauchrohrmaterial. Die federbelasteten Schraubverbindungen gestattet, die etwa bei Temperatureinwirkung auftretenden unterschiedlichen Ausdehnungen aufzufangen, damit sich die Schraubverbindungen nicht lösen können. Zur Abdeckung kann eine Abdeckplatte mit dem Basisring verbunden werden, wobei gegebenenfalls ein zylindrisches Gehäuse eingefügt werden kann, um den Raum oberhalb des offenen Tauchrohrendes mit dem Kopfteil, von dem sich Anschlussleitungen für die nichtleitende Flüssigkeit und elektrische Anschlussvorrichtungen erstrecken, zur Vermeidung von Zündquellen mit einem Inertgas wie Stickstoff, spülen zu können. Alternativ könnte dieser Raum, wie oben beschrieben, mit der nichtleitenden Flüssigkeit gefüllt werden.

Um zu verhindern, dass die Wärme, die die nichtleitende Flüssigkeit von den LEDs aufnimmt, über das Tauchrohr an die Tauchrohrumgebung bzw. das dort vorliegende Reaktionsmedium abgeleitet wird, können das Kopfteil oder der Trägerkörper oder beide Wärmeableitstrukturen wie Rippen oder Lamellen etc. aus einem wärmeleitenden Material aufweisen, die sich in den von dem Tauchrohr begrenzten Raum erstrecken, ohne die LEDs zu verschatten. D. h. Ausrichtung, Anordnung, Form und Dimensionen solcher Wärmeleitstrukturen sind so gewählt, dass die von den LEDs emittierte Strahlung nicht am Austritt aus dem Tauchrohr gehindert wird. Die Wärmeleitstrukturen können sich durch das Kopfteil aus dem Lampenmodul erstrecken, wo die Wärme an die Umgebung abgegeben werden kann.

Beim Führen der nichtleitenden Flüssigkeit ist darauf zu achten, dass die Fließgeschwindigkeit, insbesondere bei Silikonöl, gering, d. h. bevorzugt unter 1 m/s gehalten wird, da es ansonsten bei einem nicht-leitfähigen Produkt, das im Photoreaktor vorliegt, zu einer elektrostatischen Aufladung kommen kann und folglich zur Gefahr einer Zündquelle. Um den Anforderungen für eine ATEX-Zulassung bezüglich der Zündschutzart "o" = Ölkapselung zu genügen, muss die nichtleitende Flüssigkeit eine kinematische Viskosität (bei 25 °C) von zumindest 20 cSt aufweisen, auch wenn nichtleitende Flüssigkeiten mit geringeren Viskosität von beispielsweise 5 cSt technisch in Bezug auf die Kreislaufführung und Einhaltung der Oberflächentemperaturen der LEDs vorteilhafter wären.

Daher kann die nichtleitende Flüssigkeit eine kinematische Viskosität (bei 25 °C) von 5 bis 60 cSt aufweisen, wobei in Bezug auf die geltenden Normen hinsichtlich des Explosionsschutzes eine Viskosität im Bereich von 20 bis 50 cSt bevorzugt ist, um eine entsprechende Zertifizierung zu erhalten. Bei Einsatz von nichtleitenden Flüssigkeiten mit hoher Viskosität ist es ferner vorteilhaft, die Anschlussleitungen zur Vermeidung interner Druckverluste mit ausreichend großem Durchmesser zu dimensionieren, um einen zu hohen Druck an den LEDs zu vermeiden.

Um die Oberflächengrenztemperatur einzuhalten, kann das Lampenmodul in einer weiteren Ausführungsform zur Kontrolle der Fließgeschwindigkeit zusätzlich einen Durchflussmesser aufweisen, der mit einer Steuerungseinheit verbunden ist, die dazu konfiguriert ist, eine mit einer der Anschlussleitungen verbundene Fördervorrichtung und/oder Armatur in Abhängigkeit des von dem Durchflussmesser gemessenen Durchflusswerts zu steuern, um eine vorbestimmte Fließgeschwindigkeit der nichtleitenden Flüssigkeit durch den Tauchrohrinnenraum entlang einer Oberfläche der LEDs einzuhalten. Die Steuerungseinheit kann eine separate Einheit, Teil des Durchflussmessers, der Fördervorrichtung oder der Armatur oder Teil einer übergeordneten Steuerungsvorrichtung des Lampenmoduls sein. Bevorzugt können aufgrund der für den Explosionsschutz erforderlichen hohen Viskosität und der gering zu haltenden Fließgeschwindigkeit Massendurchflussmesser, beispielsweise ein Coriolis-Massendurchflussmesser oder Schwebekörperdurchflussmesser oder andere geeignete Messverfahren eingesetzt werden, wohingegen Vortexmessgeräte eher ungeeignet sind.

Die nichtleitende Flüssigkeit kann beispielsweise aus hochraffinierten Mineralölen ausgewählt werden, die praktisch ausschließlich Alkane und Cycloalkane, also gesättigte Kohlenwasserstoffe umfassen. Vorteilhaft sind Alkane und Cycloalkane vom sichtbaren Wellenlängenbereich bis in den weiten UV-C-Bereich (220-230 nm) transparent. Darunter nimmt die Transmission ab, kann aber, insbesondere bei ausreichend geringer Dicke des Spaltes zwischen den LEDs und dem Tauchrohr, noch ausreichend für Wellenlängen bis 195 nm und darunter sein. Cycloalkane können wegen des höheren Brechungsindex' im Vergleich zum entsprechenden linearen Alkan bevorzugt sein. So erstrecken sich die Brechungsindices (20°C) für C₅-C₁₄ Cycloalkane über einen Bereich von etwa 1,41 bis 1,55, während die Brechungsindices (20°C) für die korrespondierenden linearen C₅-C₁₄ Alkane einen Bereich von etwa 1,36 bis etwa 1,43 überdecken. Cyclohexan beispielsweise hat einen Brechungsindex von etwa 1,43, während Hexan einen Brechungsindex von etwa 1,37 aufweist. Ein Nachteil der gesättigten Kohlenwasserstoffe ist vor allem die Bildung leicht entzündlicher Dampf-Luft-Gemische mit einer Einordnung in Temperaturklasse 3, die für den Betrieb in entzündlichen Atmosphären eine maximale Oberflächentemperatur von 200°C festlegt. Daher ist bei Verwendung von hochraffinierten Mineralölen als nichtleitende Flüssigkeit auf einen sorgfältigen und abgedichteten Luftabschluss zu achten, um die Bildung solcher entzündlicher Dampf-Luft-Gemische zu vermeiden.

Eine bevorzugte erfindungsgemäße Ausführungsform kann als nichtleitende Flüssigkeit dünnflüssige Silikonöle vorsehen, die Brechungsindices im Bereich von etwa 1,37 bis 1,40 aufweisen, vorteilhaft nicht brennbar sind und die vom sichtbaren Wellenlängenbereich bis in den mittleren UV-C-Bereich (etwa 250 nm) transparent sind. Unterhalb von 250 nm beginnt allerdings die Transmission abzunehmen und Wellenlängen kleiner 200 nm werden absorbiert, sodass Silikonöle vor allem für Anwendungen, die Wellenlängen größer 250 nm nutzen wollen, geeignet sind. Für Anwendungen, die Wellenlängen im Bereich von 200 bis 250 nm nutzen wollen, eignen sich Silikonöle nur bedingt, nämlich wenn die Dicke des flüssigkeitsgefüllten Spalts zwischen LED und Tauchrohr und damit die Absorption klein genug ist, um eine ausreichende Transmission zu gestatten. Andernfalls sollte auf gesättigte Kohlenwasserstoffe als nichtleitende Flüssigkeit zurückgegriffen werden.

Weitere alternative nichtleitende Flüssigkeiten umfassen synthetische Ester- und Etherverbindungen. Synthetische organische Esteröle haben gegenüber den Mineralölen den Vorteil u. a. einer höheren Temperaturbeständigkeit und höheren Brenn- und Zündtemperatur und sind umweltverträglicher, weisen aber nachteilig eine geringere Alterungsbeständigkeit auf und sind höchstens bis in den mittleren UV-Bereich (etwa 270 bis 280 nm) transparent, darunter nimmt die Absorption deutlich zu. Auch bei Etherverbindungen wie beispielsweise 1,4-Dioxan mit einem Brechungsindex von 1,422 reicht die Transmission nur bis in den mittleren UV-Bereich (270 bis 300 nm, abgesehen von Diethylether bis 255 nm), allerdings nimmt die Transmission darunter weniger steil ab, sodass bei ausreichend geringer Schichtdicke zwischen LED und Tauchrohrwand Etherverbindungen als nichtleitende Flüssigkeiten auch für Wellenlängen unter 270 nm eingesetzt werden können. Wellenlängen kleiner 220 nm werden allerdings absorbiert. Hinsichtlich der Sicherheitstechnik ist aber zu berücksichtigen, dass Etherverbindungen leicht entzündliche Dampf-Luft-Gemische bilden, wobei große Unterschiede zwischen den verschiedenen Etherverbindungen bestehen. Diethylether beispielsweise fällt unter Temperaturklasse T4 (maximal zulässige Oberflächentemperatur 135°C), während 1,4-Dioxan unter Temperaturklasse 2 (maximal zulässige Oberflächentemperatur 300°C) fällt, sodass 1,4-Dioxan eher als nichtleitende Flüssigkeit eingesetzt werden kann.

Selbstverständlich kommen auch weitere Flüssigkeiten zum Einsatz in einem erfindungsgemäßen Lampenmodul in Frage, solange sie elektrisch isolierend sind und für die emittierte Wellenlänge der jeweiligen LEDs transparent sind.

Um eine für die gewünschte Transparenz erforderliche Transmission von zumindest 75 % auch bei Wellenlängen unter 250 nm bereitzustellen, kann daher ein Lampenmoduldesign vorteilhaft sein, bei dem der Innendurchmesser des Tauchrohrs in Bezug auf den Außendurchmesser des mit den LEDs bestückten Trägerkörpers so gewählt ist, dass der Spalt - und damit die Absorption - zwischen der LED-Oberfläche und der Tauchrohrinnenwand möglichst klein ist.

Um die thermisch sensiblen LEDs von den im umgebenden Reaktionsraum herrschenden Bedingungen abzukoppeln und die Funktionsfähigkeit der LEDs nicht zu beeinträchtigen, kann das Tauchrohr ein doppelwandiges Tauchrohr sein, oder das Lampenmodul kann ein zweites Tauchrohr aufweisen, in dem das erste Tauchrohr angeordnet ist. Der zwischen den beiden Tauchrohren oder den Doppelwänden gebildete Spalt stellt eine thermische Entkopplung bereit. Diese kann noch verstärkt werden, indem in dem Spalt mit einer Absaugvorrichtung ein Unterdruck erzeugt wird oder indem ein weiterer Kühlkreis zur Fluidkühlung in dem Spalt verbunden wird. Bei der Ausführung als doppelwandiges Tauchrohr kann der Spalt zwischen den Wänden auch schon bei dessen Herstellung evakuiert werden. Als Kühlfluid eignen sich alle Medien, die die emittierte Strahlung der LEDs nicht absorbieren, beispielsweise Wasser, oder Gas, insbesondere Inertgas oder auch Luft. Vorteilhaft wird durch das zusätzliche Tauchrohr die Oberfläche der Tauchlampe vergrößert und damit wird eine erhöhte photochemische Effizienz realisiert.

Vorgeschlagen wird, das bzw. die Tauchrohr(e) sinnvollerweise aus einem für die Wellenlängen der von den LEDs emittierten Strahlung durchlässigen Material zu fertigen. Zudem ist das Tauchrohr hinsichtlich Materialwahl und Formgebung auf Reaktionsdrücke im Bereich von Hochvakuum bis 6 bar Überdruck, die in dem das Tauchrohr umgebenden Reaktionsraum herrschen können, ausgelegt. Als Materialien kommen natürliches oder synthetisches Quarzglas bzw. Quarzglasmischungen oder Borsilikat, etc. in Frage. Konstruktiv kann ein Tauchrohr in gängiger Dimensionierung mit einer Wandstärke im Bereich von etwa 3,5 mm, gegebenenfalls auch darüber bis etwa 4,5 mm, ausgelegt sein. Abhängig von verschiedenen Parametern wie zum Beispiel dem verwendeten Tauchrohrmaterial, der Dimensionierung des gesamten Lampenmoduls oder einem vorgesehenen Einsatzdruck kann die Wandstärke des Tauchrohrs auch davon abweichen, d. h. die Wandstärke des Tauchrohrs wird nach den vorgegebenen Anforderungen ausgelegt. So können höhere Drücke auch Tauchrohr-Wandstärken erfordern, die größer als 4,5 mm sind, oder ein miniaturisiertes Lampenmodul kann eine Tauchrohr-Wandstärke aufweisen, die kleiner als 3,5 mm sein kann. Verwendete Tauchrohre können ferner ein- oder mehrmals getempert und druckgeprüft sein, beispielweise bis zu 10 bar.

In einer weiteren Ausführungsform kann der Trägerkörper zumindest eine innenliegende Kammer aufweisen, die durch den Trägerkörper begrenzt wird, sodass sich durch die Kammer zumindest eine Stromversorgungs- und/oder Steuerungsleitung von dem Kopfende des Trägerkörpers bis zu einem Kontaktelement der zumindest einen LED erstrecken kann. Das Kontaktelement kann beispielsweise in einer Ausnehmung in der Wand des Trägerkörpers angeordnet sein, die die Kammer begrenzt, sodass die Kontaktierung mit der Stromversorgungs- und/oder Steuerungsleitung von innen erfolgen kann. Falls aber das Kontaktelement an der Außenseite des Trägerkörpers angeordnet ist, kann die Kammer zur Kontaktierung mit der Stromversorgungs- und/oder Steuerungsleitung zumindest eine Öffnung zur Außenseite des Trägerkörpers aufweisen, durch die die zumindest eine Stromversorgungs- und/oder Steuerungsleitung durchtreten kann. Der Eintritt der nichtleitenden Flüssigkeit durch diese Öffnung in die Kammer ist dabei gestattet, sodass hierdurch ein zusätzlicher Kühlungseffekt eintritt. Alternativ kann diese Öffnung um die durchtretende Stromversorgungs- und/oder Steuerungsleitung abgedichtet sein, um den Eintritt der nichtleitenden Flüssigkeit in die Kammer zu vermeiden. Bevorzugt können sämtliche elektrischen Verbindungen, die von der nichtleitenden Flüssigkeit umgeben sind oder damit in Kontakt kommen, fluiddicht ausgeführt sein, um zu vermeiden, dass die nichtleitende Flüssigkeit infolge von Kriech- und Kapillareffekten aufgrund der Oberflächenspannung beispielsweise zwischen Kontaktierungsstellen, wo unter Umständen der elektrische Kontakt verschlechtert oder unterbrochen wird. Bei Steckverbindungen können abgedichtete Stecker unter Umständen ausreichen, gegebenenfalls sind aber auch weitere Maßnahmen, z. B. ein Verlöten der Kontaktstellen erforderlich, um nicht nur um ein Kriechen der nichtleitenden Flüssigkeit zu vermeiden, sondern auch den elektrischen Kontakt sicherzustellen.

An dem Kopfteil des Lampenmoduls können elektrische und mechanische Anschlussvorrichtungen vorgesehen sein. Elektrische Anschlussvorrichtungen sorgen für die Verbindung der Stromversorgungs- und/oder Steuerungsleitung mit einer Stromversorgungs- und Steuerungsvorrichtung des Lampenmoduls. Die Stromversorgungs- und Steuerungsvorrichtung des Lampenmoduls für die LEDs, die beispielsweise Vorschalt- bzw. Leistungselektronik, Treiber und Netzteile umfassen kann, kann eine externe Stromversorgungs- und Steuerungsvorrichtung sein, die über eine erste elektrische Anschlussvorrichtung des Kopfteils angeschlossen wird. Alternativ kann die Stromversorgungs- und Steuerungsvorrichtung des Lampenmoduls gegebenenfalls im Kopfteil untergebracht sein - dann dient die elektrische Anschlussvorrichtung der Verbindung mit einer Stromquelle. Über eine weitere elektrische Anschlussvorrichtung wird dann die Stromversorgungs- und/oder Steuerungsleitung am Kopfteil angeschlossen.

Elektrische Anschlüsse durch das Kopfteil werden, um zu vermeiden, dass die nichtleitende Flüssigkeit durch die Leitungen aus dem Kopfteil in den Raum oberhalb des offenen Tauchrohrendes gelangt, vorzugsweise längswasserdicht (bzw. fluiddicht) ausgeführt und können beispielsweise bei der Herstellung des Kopfteils damit vergossen werden, sodass keine Flüssigkeit aufgrund von Kapillareffekten durch die Isolation oder den Schirm der elektrischen Leitung nach außen dringen kann. Bevorzugt können auch hier die Verbindungsstellen verlötet werden.

Zur Kühlung einer im Kopfteil untergebrachten Stromversorgungs- und Steuerungsvorrichtung können die sich durch das Kopfteil erstreckenden Anschlussleitungen eingesetzt werden und dann einen oder mehrere Kühlmittelpfad(e) aufweisen.

Für den mechanischen Halt des Lampenmoduls sorgt eine mechanische Anschlussvorrichtung zur Verbindung des Kopfteils mit einer entsprechenden Halterung. Sämtliche Anschlussvorrichtungen können bevorzugt als lösbare Steck-, Schraub-, Steckschraub-, Klemmverbindungen oder ähnliches ausgeführt sein, sodass das Lampenmodul einfach von der Stromversorgungs- und/oder Steuerungsleitung bzw. -vorrichtung, den Kühlmittelleitungen und der mechanischen Halterung getrennt und wieder angefügt werden kann. Halterung und Kühlmittelleitung können je nach Anordnung des Lampenmoduls im Reaktor (horizontal oder vertikal) als starre (Rohr-)Verbindungen (beispielsweise im Falle horizontaler Anordnung) ausgeführt sein oder es werden nicht-starre Schläuche für die Kühlmittelleitungen und Metall- oder Kunststoffseile oder Ketten für die Halterung (beispielsweise im Falle vertikaler Anordnung) eingesetzt, sodass die LED-Lampe quasi frei schwingend am Kopfteil aufgehängt ist, was die Handhabung des Lampenmoduls erleichtert.

Des Weiteren kann das Lampenmodul eine Mehrzahl von LEDs aufweisen, wobei jeweils eine Teilanzahl der Mehrzahl der LEDs auf jeweils einer Trägerplatine angeordnet ist und die Trägerplatinen an dem Trägerkörper befestigt sind. Dies ermöglicht, dass bei Ausfall einer LED auf einer Platine nicht alle LEDs abgeschaltet werden müssen, sondern lediglich die betroffene Platine, während die anderen Platinen weiter in Betrieb bleiben können, sodass der Austausch der betroffene Platine warten kann, bis eine Reaktion im umgebenden Reaktor durchgeführt wurde.

Dazu kann das Lampenmodul pro Platine eine Erkennungseinheit aufweisen, die dazu konfiguriert ist, einen Ausfall einer oder mehrerer LEDs auf der Trägerplatinen festzustellen und in Abhängigkeit eines festgestellten Ausfalls die Stromversorgung für die betroffene Platine zu unterbrechen und ggf. für die weiteren Trägerplatinen korrespondierend zu begrenzen. Ggf. kann über die Steuerungsvorrichtung des Lampenmoduls auch eine Warnmeldung ausgegeben werden, wenn die Erkennungseinheit mit der Stromversorgungs- und Steuerungsvorrichtung des Lampenmoduls verbunden ist. Grundsätzlich ist eine solche Erkennungseinheit auch pro LED denkbar, sodass bei Ausfall einzelner LEDs die jeweilige Stromversorgung unterbrochen und die Stromversorgung für die weiteren LEDs ggf. korrespondierend begrenzt wird.

Ferner kann das Lampenmodul einen oder mehrere Temperatursensor(en) aufweisen, der/die auf dem Trägerkörper angeordnet und mit der Stromversorgungs- und Steuerungsvorrichtung des Lampenmoduls, die einen Schutzschalter für LEDs umfasst, verbunden ist/sind. Die Stromversorgungs- und Steuerungsvorrichtung kann ferner alternativ oder zusätzlich zumindest einen Regelkreis für die LED-Ansteuerung aufweisen, mit dem gleichartige oder unterschiedliche LEDs dimmbar sind und/oder das Spektrum der emittierten Wellenlängen unterschiedlicher LEDs änderbar ist, um die emittierte Lichtmenge oder die emittierten Wellenlängen dem Reaktionsverlauf im umgebenden Reaktionsraum anzupassen.

Eine weitere Ausführungsform des erfindungsgemäßen Lampenmoduls kann vorsehen, dass in einer der im Kreislauf geführten Anschlussleitungen, vorzugsweise an einer höchstgelegenen Stelle, eine Atmungseinheit mit Trocknungsmitteln vorgesehen ist, um zur Vermeidung von Kondenswasser eine Entlüftung der nichtleitenden Flüssigkeit zu ermöglichen, wobei ein Eintrag von Feuchtigkeit durch Frischluft durch ein Trocknungsmittel wie beispielsweise Silicagel vermieden wird.

Gemäß einer weiteren Ausführungsform des Lampenmoduls kann zumindest eine, bevorzugt alle LEDs des Lampenmoduls, ohne Primäroptik ausgeführt sein. Durch die vorliegende nichtleitende Flüssigkeit ist die LED bereits ausreichend vor Umwelteinflüssen geschützt, sodass auf die Primäroptiken, die abhängig von den Einsatzbedingungen einem gewissen Alterungsprozess unterliegen, gänzlich verzichtet werden kann.

Ein erfindungsgemäßer Photoreaktor weist ein Reaktionsgefäß auf, das einen Reaktionsraum zur Durchführung einer photochemischen Reaktion zumindest eines in das Reaktionsgefäß zugeführten oder dort vorgelegten Edukts begrenzt. Ferner umfasst er zumindest ein in dem Reaktionsgefäß angeordnetes erfindungsgemäßes Lampenmodul mit einem für die photochemische Reaktion geeigneten Emissionsspektrum.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung.

Dabei zeigen:
- **Fig. 1**: eine schematische Seitenansicht einer ersten Ausführungsform eines erfindungsgemäßen LED-Lampenmoduls,
- **Fig. 2**: eine schematische Seitenansicht einer weiteren Ausführungsform eines erfindungsgemäßen LED-Lampenmoduls,
- **Fig. 3**: eine schematische Seitenansicht einer weiteren Ausführungsform eines erfindungsgemäßen LED-Lampenmoduls,
- **Fig. 4**: eine schematische Seitenansicht eines Trägerkörpers mit Kopfteil einer weiteren Ausführungsform eines erfindungsgemäßen LED-Lampenmoduls,
- **Fig. 5**: eine schematische Querschnittansicht einer weiteren Ausführungsform eines erfindungsgemäßen LED-Lampenmoduls,
- **Fig. 6**: eine schematische Draufsicht auf eine Trägerplatine mit LEDs und Kontaktelement,
- **Fig. 7**: eine schematische Querschnittansicht einer weiteren Ausführungsform eines erfindungsgemäßen LED-Lampenmoduls,
- **Fig. 8**: eine schematische Querschnittansicht durch eine herkömmliche LED (a) und eine primäroptiklose LED (b) eines Lampenmoduls nach einer erfindungsgemäßen Ausführungsform,
- **Fig. 9**: eine schematische Seitenansicht einer weiteren Ausführungsform eines erfindungsgemäßen LED-Lampenmoduls,
- **Fig. 10**: eine schematische Seitenansicht eines Kopfteils und Trägerkörpers nach einer weiteren Ausführungsform eines erfindungsgemäßen LED-Lampenmoduls,
- **Fig. 11**: eine perspektivische Detailansicht des Kopfteils aus Fig. 1 in der abdichtenden Anordnung am offenen Ende eines Tauchrohrs nach einer weiteren Ausführungsform eines erfindungsgemäßen LED-Lampenmoduls,
- **Fig. 12**: eine perspektivische Detailansicht eines in einem Basisring angeordneten Tauchrohrs,
- **Fig. 13**: eine perspektivische Detailansicht eines auf Fig. 12 folgenden Montageschritts, in dem das Tauchrohr durch einen Anpressring am Basisring befestigt wird,
- **Fig. 14**: eine perspektivische Detailansicht einer zu Fig. 11 alternativen Befestigung des Kopfteils an dem Tauchrohr,
- **Fig. 15**: eine Seitenansicht eines erfindungsgemäßen LED-Lampenmoduls mit den Ausführungen aus Fig. 9 bis 13.

Die erfindungsgemäße Vorrichtung ist ein LED-Lampenmodul, das dazu ausgebildet ist, als Tauchstrahler in einem Photoreaktor eingesetzt zu werden, und ein entsprechender Photoreaktor, der mit einem oder mehreren erfindungsgemäßen LED-Lampenmodulen ausgestattet ist.

Das erfindungsgemäße LED-Lampenmodul ist primär zur industriellen Anwendung in der präparativen Photochemie vorgesehen, und muss daher den erhöhten Anforderungen genügen. Hierzu zählen insbesondere von Raumtemperatur und Umgebungsdruck abweichende Reaktionstemperaturen und -drücke, die im Reaktionsraum um das Lampenmodul herrschen, und die auch Temperaturen unter + 5 °C und über + 40 °C sowie Drücke im Bereich von Hochvakuum und 6 bar Überdruck umfassen. Ferner ist bei den in Photoreaktoren eingesetzten Lichtquellen - je nach Zusammensetzung des Reaktionsvolumens - auf den Explosionsschutz zu achten; die erfindungsgemäßen LED-Lampenmodule können in bestimmten Ausführungsformen auch in ATEX klassifizierten Bereichen eingesetzt werden.

Für die ATEX-Zulassung ist in DIN EN 60079-6 eine Spezifikation für Isolierflüssigkeiten vorgeschrieben, wofür eine nichtleitende Flüssigkeit mit einer Viskosität größer oder gleich 20 cSt, beispielsweise 50 cSt, gewählt werden muss. Geringere Viskositäten sind für ein erfindungsgemäßes Lampenmodul ebenfalls umsetzbar, sofern die Anforderungen der Norm nicht erfüllt sein müssen.

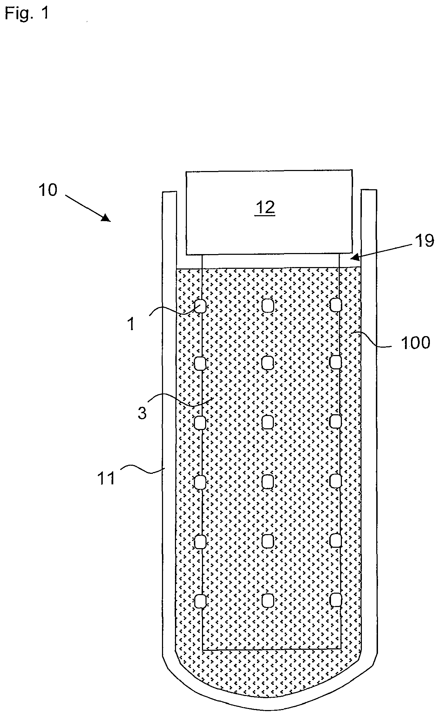

**Fig. 1** zeigt beispielhaft ein Lampenmodul 10, das als Tauchlampe zur vertikalen Anordnung in photochemische Reaktoren konzipiert ist. Das Lampenmodul 10 weist einen Trägerkörper 3 auf, an dessen Mantelfläche Licht emittierenden Dioden (LEDs) 1 verteilt angeordnet sind. Der Trägerkörper 3 ist mit einem Kopfteil 12 verbunden, das einen elektrischen Anschluss der LEDs 1 bereitstellt und zur Halterung des Trägerkörpers 3 dient. Ferner weist das Lampenmodul 10 ein Tauchrohr 11 aus einem für die Wellenlängen der von den LEDs 1 emittierten Strahlung durchlässigen Material auf, das einen Tauchrohrinnenraum 19 begrenzt, in dem der Trägerkörper 3 mit den LEDs 1 angeordnet ist. Dieser Tauchrohrinnenraum 19 ist mit einer nichtleitenden Flüssigkeit 100 gefüllt, sodass die LEDs 1 vollständig eingetaucht sind. Die nichtleitenden Flüssigkeit 100, die aus gesättigten Kohlenwasserstoffen, Silikonölen und synthetischen Ester- und Etherverbindungen ausgewählt ist, ist dabei für die Wellenlängen der von den LEDs 1 emittierten Strahlung transparent und weist einen Brechungsindex (20°C) im Bereich von zumindest 1,35 bis etwa 1,45, ggf. auch bis etwa 1,55 auf. Dadurch wird sowohl die Photonen-Auskopplungseffizienz aus jeder LED 1 vergrößert, da weniger Photonen an der Grenzschicht zwischen Diodensubstrat und nichtleitender Flüssigkeit reflektiert werden, als auch die Reflexion an der Phasengrenze zwischen nichtleitender Flüssigkeit 100 und Tauchrohrwand 11 verringert, sodass die Gesamtlichtleistung des Lampenmoduls 10, d. h. die Strahlungsmenge und -dichte an der Außenfläche des Tauchrohrs 11, deutlich erhöht ist. Ferner trägt die nichtleitende Flüssigkeit zur Kühlung der LEDs 1 und damit zur Verlängerung deren Lebensdauer bei.

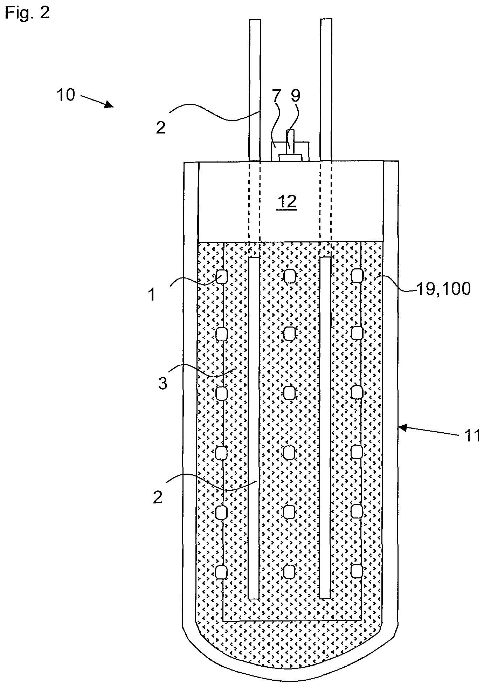

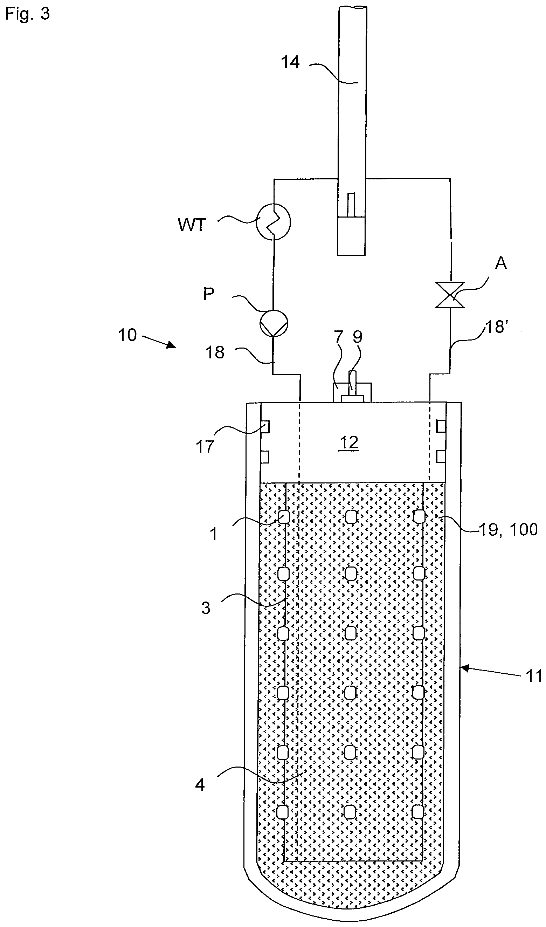

Die Kühlungseffizienz durch die nichtleitende Flüssigkeit kann durch Wärmeleitstrukturen an Kopfteil und/oder Trägerkörper verbessert werden. **Fig. 2** zeigt rippenartige Wärmeleitstrukturen 2, die entlang dem Trägerkörper 3 zwischen vertikalen Reihen von LEDs 1 angeordnet sind und die Wärme aus der nichtleitenden Flüssigkeit 100 aufnehmen können. Die Wärmeleitstrukturen 2 sind hierbei durch das Kopfteil 12 nach außen geführt, um dort die abgeleitete Wärme an die Umgebung abzugeben. Das Kopfteil 12 weist ferner eine elektrische Anschlussvorrichtung 7 und eine mechanische Anschlussvorrichtung 9 auf und ist in dem offenen Ende des Tauchrohrs 11 angeordnet. Eine entsprechende Halterung 14 zur Verbindung mit der mechanischen Anschlussvorrichtung 9 ist in **Fig. 3** dargestellt.

Nicht dargestellt in **Fig. 1** **und** **2** sind die Anschlussleitungen zur Kreislaufführung der nichtleitenden Flüssigkeit, um die von den LEDs 1 und von dem Reaktionsmedium in der Tauchrohrumgebung aufgenommene Wärme außerhalb des Tauchrohrs 11 abzuführen.

Zur Abdichtung des Tauchrohrinnenraums 19 kann das Kopfteil 12 dicht mit dem offenen Ende des Tauchrohrs 11 verbunden sein, wie dies in **Fig. 3** durch die Dichtungen 17 angedeutet ist. Selbstverständlich sind Abweichungen in Form, Anordnung und Dichtkonzept des Kopfteils an dem Tauchrohr von dem dargestellten Beispiel möglich und vom Schutzumfang umfasst. So kann beispielsweise das Kopfteil über das Tauchrohr auch hinausragen und die Dichtung kann über die Stirnfläche oder den Außenumfang des Tauchrohrs erfolgen.

Eine Ausführung mit einer federgelagerten Verbindung des Kopfteils mit dem Tauchrohr zur Verbesserung der mechanischen Stabilität des Lampenmoduls als Tauchlampe ist weiter unten im Zusammenhang mit **Fig, 11 bis 14** ausgeführt. Dazu weist das Kopfteil einen Kragen auf, auf dem eine Ringscheibe angeordnet wird, die über Federschrauben mit einem Ringflansch verbunden wird, der am offenen Ende des Tauchrohrs angeordnet ist. Dazu kann der Endabschnitt des Tauchrohrs nach oben konusförmig aufgeweitet sein und der Ringflansch eine entsprechende konusförmige Öffnung aufweisen.

Ferner zeigt **Fig. 3** eine Kreislaufführung der nichtleitenden Flüssigkeit 100, die durch die Wirkung der Pumpe P über eine Anschlussleitung 18, die sich durch das Kopfteil 12 erstreckt und mit dem Zulaufabschnitt 4 verbunden ist, der sich durch den Trägerkörper 3 erstreckt, am unteren Ende in den Tauchrohrinnenraum 19 zugeführt und über die Anschlussleitung 18', die sich durch das Kopfteil 12 erstreckt, am oberen Ende des Tauchrohrinnenraums 19 ausgeleitet wird. Ein Wärmetauscher WT ermöglicht die Ableitung der von der nichtleitenden Flüssigkeit 100 aufgenommenen Wärme. Mittels der optionalen Armatur A kann gegebenenfalls ein Druck im Tauchrohrinnenraum 19 angepasst werden.

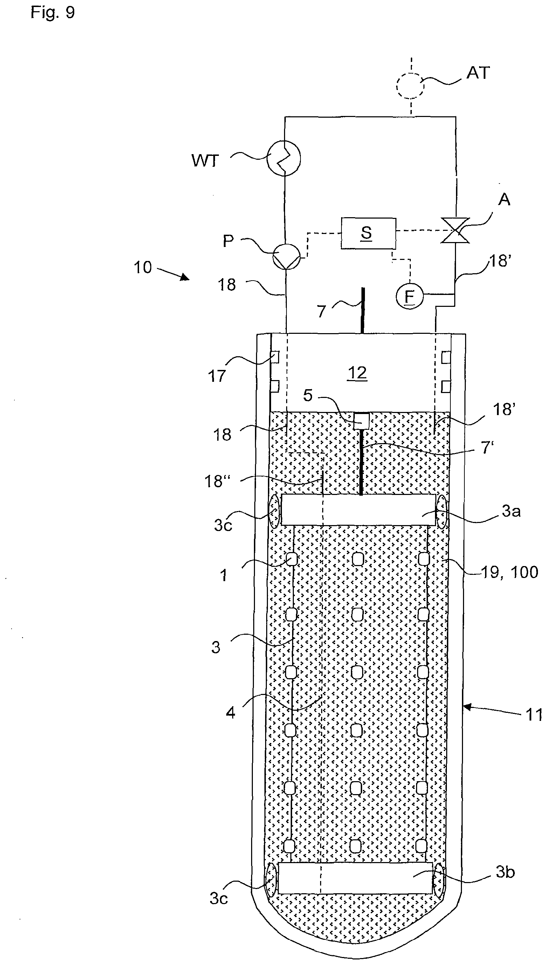

Zur Einhaltung der Oberflächengrenztemperatur des Lampenmoduls 10 während des Betriebs ist es sinnvoll, das Fließvolumen der im Kreislauf geführten nichtleitenden Flüssigkeit 100, das einen vorbestimmten Mindestwert nicht unterschreiten sollte, zu überwachen, wofür ein oder mehrere Durchflussmesser im Kreislauf angeordnet sein können. **Fig. 9** zeigt beispielhaft einen Durchflussmesser F an der Anschlussleitung 18', der mit einer Steuerungseinheit S verbunden ist, die zur Kontrolle der Fließgeschwindigkeit der nichtleitenden Flüssigkeit 100 Pumpe P oder vorzugsweise Armatur A, gegebenenfalls auch beide, steuert. Für die zur ATEX-Zertifizierung erforderlichen hohen Viskositäten und geringe Fließgeschwindigkeiten sind als Durchflussmesser Coriolis-Massendurchflussmesser geeignet, die die Sicherheitsstufe ("safety integrity level", kurz SIL) für eine ATEX-Zulassung erfüllen. Alternativ sind Schwebekörperdurchflussmesser einsetzbar, die ebenfalls auch bei hoher Viskosität und geringer Fließgeschwindigkeit der nichtleitenden Flüssigkeit 100 zuverlässige Messwerte liefern, wohingegen Vortex-Durchflussmesser weniger geeignet sind.

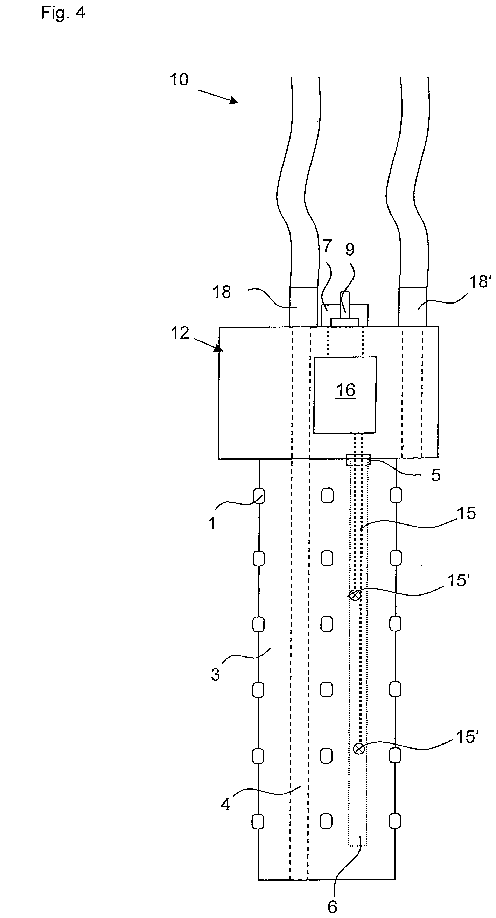

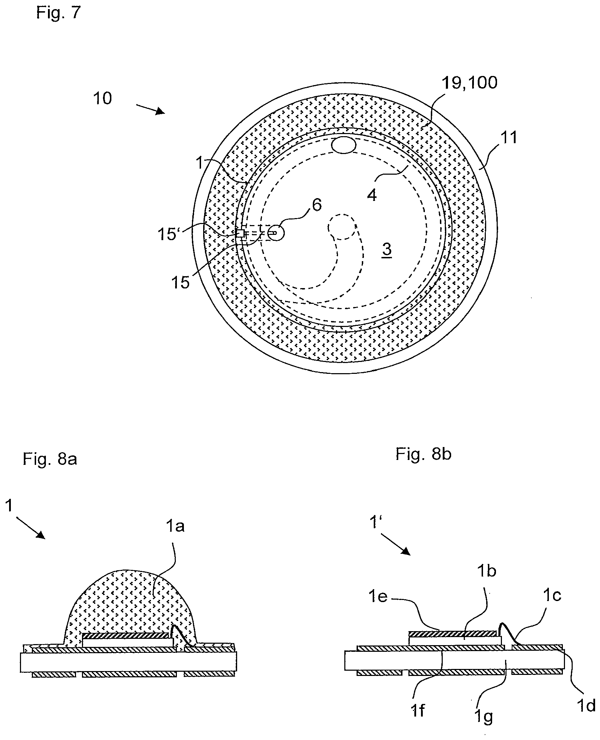

**Fig. 4** zeigt Ausführungsvarianten des Trägerkörpers 3 und des Kopfteils 12. Der als Kühlkörper ausgebildete Trägerkörper 12 begrenzt mit Zufuhrabschnitt 4 den internen Fluidpfad für die nichtleitende Flüssigkeit 100. Dadurch werden die LED(s) 1, bzw. deren Träger- oder Kühlstrukturen 1g (vgl. **Fig. 8****)** quasi von der Rückseite gekühlt. Der Zufuhrabschnitt 4 mündet an dem Kopfteil abgewandten Ende des Trägerkörpers 3 in den Fußraum des Tauchrohrs 11 und strömt von dort außen an der Oberfläche des Trägerkörpers 3 mit den LEDs entlang nach oben, um durch die Anschlussleitung 18' abgeführt zu werden. Anders als in **Fig. 4** dargestellt, können auch mehr als ein Fluidpfad durch den Kühlkörper 3 geführt werden um eine optimale Kühlung der LEDs 1 zu erreichen. Dadurch, dass die aktive Kühlung mit der nichtleitenden Flüssigkeit den Einsatz von Modulen mit einer Vielzahl an LEDs bzw. den Betrieb der LEDs mit hoher Leistung erlaubt, wird eine hohe Leistungsdichte erreicht, die mit Nieder- und Mitteldruckstrahlern im Bereich der Photochemie konkurrieren kann. Das Handhaben und Beherrschen des Wärmehaushalts - kurz thermisches Management - eines LED-Lampenmoduls, das auch die Prozesstemperatur des Reaktionsmediums, das das Lampenmodul umgibt, berücksichtigen muss, ist für eine adäquate Lebensdauer von entscheidender Bedeutung.

Die Abschnitte der Anschlussleitungen 18, 18' im Kopfteil 12 dienen dabei gleichzeitig der Kühlung der Stromversorgungs- und Steuerungsvorrichtung 16, die in diesem Beispiel im Kopfteil 12 untergebracht ist und einerseits mit der elektrischen Anschlussvorrichtung 7 und andererseits mit den Stromversorgungs- und/oder Steuerungsleitungen 15 verbunden ist, die sich durch die Kammer 6, die sich im Trägerkörper 3 befindet, zum Anschluss mit Kontaktelementen 15' der LEDs 1 auf unterschiedlicher Höhe erstrecken.

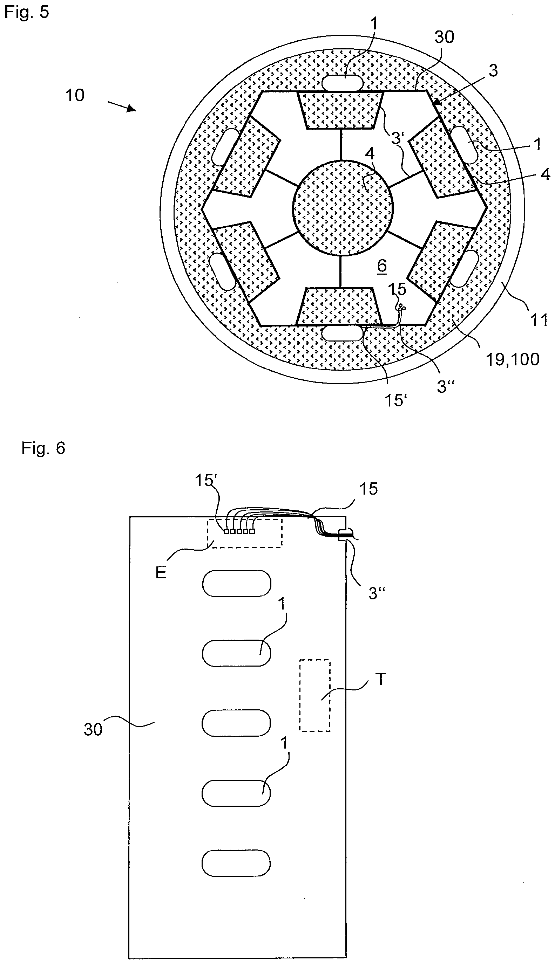

Die Kammer 6 und Kontaktelemente 15' sind auch in den Beispielen aus **Fig. 5 und 6** zu sehen, wobei **Fig. 5** ein LED-Lampenmodul 10 zeigt, dessen Trägerkörper 3 durch ein sechseckiges Hohlprofil, beispielsweise aus Aluminium mittels Strangpressen, als Kühlkörper mit innenliegenden Zufuhrkanälen 4 ausgebildet ist. Die Zulaufkanäle 4 sind dabei über Stege 3' verbunden, sodass die Zwischenräume als Kabelkammern 6 zur Verfügung stehen. Durch eine Öffnung 3" in der Außenwand des Trägerkörpers 3 werden die Stromversorgungs- und/oder Steuerungsleitung 15 aus der Kammer 6 nach außen zu den Kontaktelementen 15' geführt, die hier zur Versorgung der auf einer Platine 30 angeordneten LEDs 1 gemeinsam an deren Ende neben der Öffnung 3" angeordnet sind. Sämtliche elektrischen Kontakte können verlötet sein, um ein Eindringen der nichtleitenden Flüssigkeit zu vermeiden. Auch die Öffnung 3" kann um die Stromversorgungs- und/oder Steuerungsleitung 15 verschlossen, z. B. verlötet werden, um ein Eindringen der nichtleitenden Flüssigkeit in Kammer 6 zu vermeiden. Alternativ oder zusätzlich kann die Verbindung 5 der Stromversorgungs- und/oder Steuerungsleitung 15 zum Kopfteil 12 (vgl. **Fig. 4****)** längsfluiddicht ausgeführt sein, sodass das Fluten der Kammer 6 mit der nichtleitenden Flüssigkeit gestattet sein kann.

Da der Abstrahlwinkel herkömmlicher LEDs beschränkt ist, sind mehreckige, z. B. sechs- oder achteckige, Profilquerschnitte des Trägerkörpers vorteilhaft. Werden OLEDs eingesetzt, können auch kreiszylindrische Formen gut realisiert werden.

**Fig. 7** zeigt eine OLED 1, die hier als Schichtverbund auf dem zylindrischer Trägerkörper 3 angeordnet und in die nichtleitende Flüssigkeit 100 im Tauchrohr 11 getaucht ist. Auch dieser Trägerkörper 3 weist einen Kühlfluidpfad auf, der hier allerdings mit einem helixförmigen Zulaufabschnitt 4 ausgebildet ist, der kopfteilseitig eine seitliche Öffnung zur Verbindung mit einer nicht dargestellten Anschlussleitung aufweist und mittig am Boden des Trägerkörpers 3 in der gestrichelt dargestellten unbezeichneten Öffnung mündet. Zum Anschluss der OLED 1 an dessen Kontaktelement 15' erstreckt sich auch hier eine Stromversorgungs- und/oder Steuerungsleitung 15 durch eine Kammer 6 innerhalb des Trägerkörpers 3.

Grundsätzlich können alle Verbindungsstellen, z. B. an den Kontaktelementen 15', der Zugang 3" zu den Kabelkammern 6 und an einer elektrischen Verbindung 5 am Kopfteil 12 (siehe z. B. **Fig. 4****, 9)** längswasserdicht ausgeführt werden, um zu verhindern, dass Teile der nichtleitenden Flüssigkeit 100 in die Kabelkammern 6 und weiter durch Kapillareffekte entlang elektrischer Leitungen durch das Kopfteil 12 nach außerhalb des Lampenmoduls 10 dringen. Hierzu können Maßnahme, wie beispielsweise das Eingießen von Leitungsabschnitten in das Kopfteil und/oder mittels Dichtungen abgedichtete Steckverbindungen gegebenenfalls ausreichen. Um aber das Eindringen der nichtleitenden Flüssigkeit, die bei elektrischen Steckverbindungen gegebenenfalls auch zur Unterbrechung der Kontaktierung führen kann, sicher zu verhindern, können die Kontaktstellen beispielsweise durch Verlöten abgedichtet werden.

In **Fig. 8** ist a) eine herkömmliche LED 1 mit einer Kunststofflinse als Primäroptik 1a dargestellt, die in einem erfindungsgemäßen LED-Lampenmodul 10 zum Einsatz kommen kann. **Fig. 8b****)** zeigt eine primäroptiklose LED 1', deren Aufbau ansonsten mit Halbleiterkristall 1b, Draht 1c, Anode 1d, LED-Chip 1e, Kathode 1f und Träger 1g der herkömmlichen LED 1 aus **Fig. 8a****)** entspricht, und die in einem erfindungsgemäßen LED-Lampenmodul 10 eingesetzt werden kann, da dort die nichtleitende Flüssigkeit 100 die Funktionen der Primäroptik übernimmt. Vorteilhaft kann hierdurch abgesehen von der Einsparung eines Bauteils der Kühleffekt weiter verbessert werden.

**Fig. 9** zeigt schematisch eine Ausführungsform des erfindungsgemäßen Lampenmoduls 10, bei dem der Trägerkörper 3 mit den LEDs 1 nicht direkt mit dem Kopfteil 12 verbunden ist. Der Trägerkörper 3 mit den LEDs 1 weist hier beidseitig jeweils einen Sockelabschnitt 3a, 3b auf, der mittels Ringfeder 3c die zentrierte Anordnung im Tauchrohr 11 erleichtert. Die Ringfeder 3c gestattet den Durchtritt der nichtleitenden Flüssigkeit 100 am Sockel 3a, 3b vorbei. Grundsätzlich ist der kopfteilnahe Sockelabschnitt 3a mit einem Anschlussleitungsabschnitt 18" ausgestattet, der sich von der dem Kopfteil 12 zugewandten Seite des Sockelabschnitts 3a durch den Sockelabschnitt 3a zum Zufuhrabschnitt 4 im Trägerkörpers 3 erstreckt. Der Zufuhrabschnitt 4 - selbstverständlich kann der Anschlussleitungsabschnitt 18" auch mit einem Verteiler auf mehrere Zufuhrabschnitte verbunden sein - mündet auf der vom Kopfteil 12 abgewandten Seite des kopfteilfernen Sockelabschnitts 3b. Der Anschlussleitungsabschnitt 18" wird mit der zuleitenden Anschlussleitung 18 verbunden, während sich die ableitende Anschlussleitung 18' am Kopfteil 12 zum Innenraum 19 öffnet.

Ferner weist der obere Sockelabschnitt 3a eine elektrische/elektronische Anschlussvorrichtung 7' zur Stromversorgung und Steuerung der LEDs 1 auf, die über einen längswasserabgedichteten Anschluss 5 mit der Anschlussvorrichtung 7 des Kopfteils 12 verbunden ist, das mittels Dichtungen 17 abgedichtet im offenen Ende des Tauchrohrs 11 angeordnet ist.

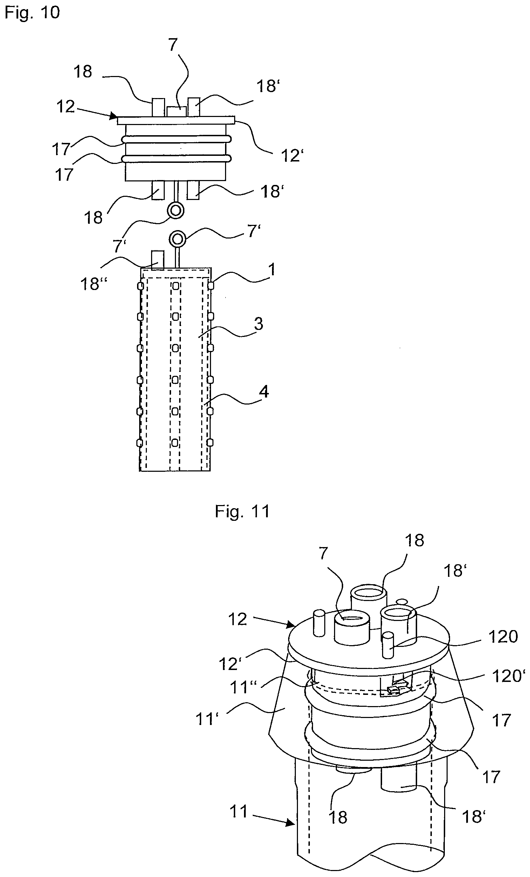

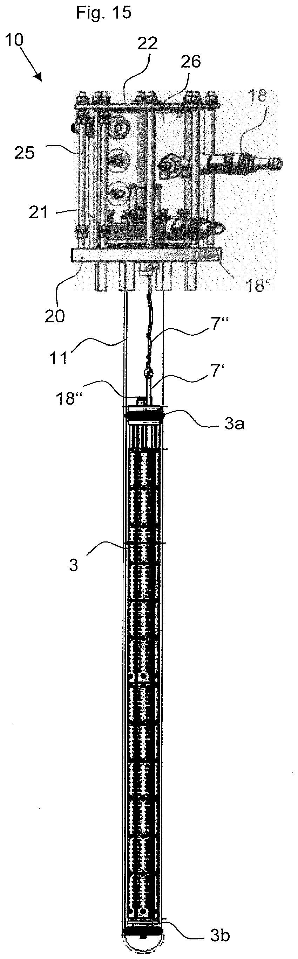

Wie in **Fig. 10** skizziert ist, können zur elektrischen/elektronischen Kontaktierung eines mit LEDs 1 bestückten Trägerkörpers 3 mit einem separaten Kopfteil 12 sowohl der Trägerkörper 3 (bzw. ein in **Fig. 10** nicht dargestellter Sockelabschnitt 3a) als auch das Kopfteil 12 einander zugewandte Anschlussvorrichtungen 7' aufweisen, die, wie in **Fig. 15** zu sehen ist, mit einem zusätzlichen Verbindungselement 7", hier in Form einer Kette, verbunden werden, die eine relative Beweglichkeit zwischen Trägerkörper 3 und Kopfteil 12 zulässt, und damit Spannungen, die beispielsweise infolge von thermischen Ausdehnungseffekten auftreten können, vermeidet.

In **Fig. 10** sind ferner die Anschlussleitungen 18, 18' am Kopfteil 12 zur Zu- und Ableitung der nichtleitenden Flüssigkeit, zwei am Kopfteil 12 axial beabstandete Dichtringe 17 und ein Flanschabschnitt 12' zu sehen, der das Kopfteil 12 auf der vom Trägerkörper 3 weg weisenden Seite abschließt und zur abdichtenden Anordnung am offenen Ende des Tauchrohrs 11 beiträgt, wie im Beispiel von **Fig. 11** dargestellt ist. Der Trägerkörper 3 weist hier mehrere Zufuhrabschnitte 4 auf, die sich vom Anschlussleitungsabschnitt 18" erstrecken, der auch hier über einen nicht dargestellten Leitungsabschnitt mit der zuleitenden Anschlussleitung 18 zu verbinden ist. Die Anordnung der Zulaufabschnitte 4 korrespondiert mit der Anordnung der LEDs 1 am Trägerkörper 3, um eine optimale und gleichmäßige rückseitige Kühlung der LEDs 1 zu erreichen.

Das offene Ende des Tauchrohrs 11 ist in **Fig. 11** mit einem sich vom offenen Ende aus aufweitenden, konusförmigen Kragen 11' ausgebildet, in dem das Kopfteil 12 aufgenommen ist. Sofern das Kopfteil 12 wie in dem dargestellten Beispiel einen Flanschabschnitt 12' aufweist, kann dieser entweder wie in **Fig. 11** auf der Stirnseite des offenen Endes des Tauchrohrs 11 oder auf einem nicht abgebildeten Ringabsatz aufliegen, der an der Tauchrohrinnenwand ausgebildet ist. Zur Fixierung des Kopfteils 12 und Sicherung der Abdichtung durch die Dichtringe 17, die gegen die Tauchrohrinnenwand gepresst werden, weist das Kopfteil 12 Schwenkriegel 120 auf, deren Flügelabschnitt 120' durch Drehung des Schwenkriegels 120 in eine Ringnut 11' eingreifen, die an der Tauchrohrinnenwand ausgebildet ist.

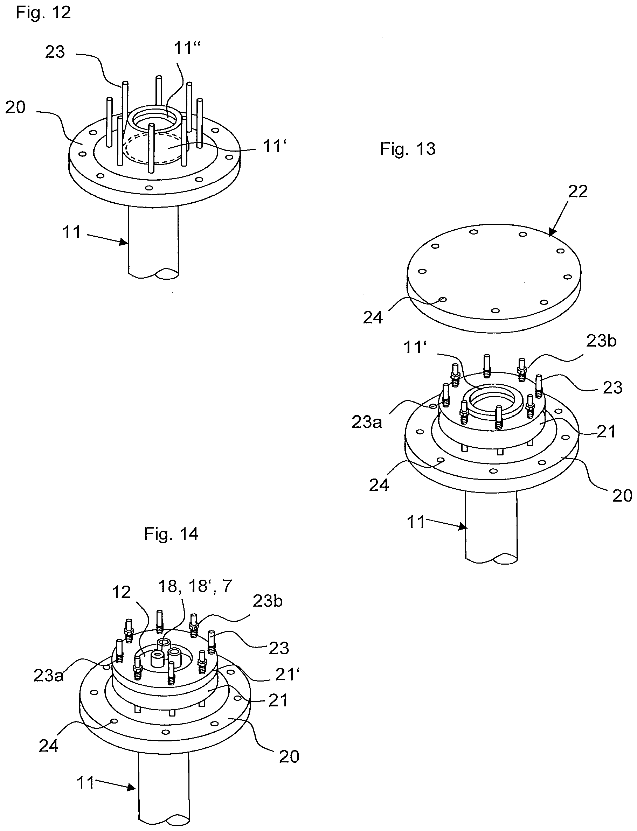

Alternativ zur Befestigung mit Schwenkriegeln 120, die eine punktuelle Befestigung bereitstellen, kann bevorzugt ein Anpressring 21' vorgesehen sein, wie im Beispiel von **Fig. 14** skizziert ist, der eine Öffnung mit einem Durchmesser aufweist, der kleiner ist als der Durchmesser des Kopfteils 12, sodass der Anpressring 21' umfänglich flächig mit einem Ringabschnitt auf dem Kopfteil 12 aufliegen und dieses im offenen Ende des Tauchrohrs 11 halten kann, wenn der Anpressring 21' mit dem Haltering 21 für das Tauchrohr 11 an einer Basisplatte 20 befestigt wird, was im Folgenden beschrieben wird. Der Zugriff zu den Anschlussleitungen 18, 18' und der elektrischen Anschlussvorrichtung 7 bleibt durch die Öffnung des Anpressrings 21' erhalten.

Um eine LED-Lampe 10, wie in **Fig. 15** gezeigt, gebrauchsfertig bereitzustellen, etwa zum Einsatz in einem (nicht dargestellten) Reaktor, kann das Tauchrohr 11 (vgl. **Fig. 12****),** das einen konusförmigen Kragen 11' aufweist, in einer Öffnung einer Basisplatte 20 angeordnet werden, wobei der Durchmesser der Öffnung auf den Außendurchmesser des Tauchrohrs 11 angepasst ist, sodass der Kragen 11' des Tauchrohrs 11 auf der Basisplatte 20 aufliegt. In **Fig. 12** bis **14** ist die Basisplatte 20 als Basisring 20 ausgebildet, aber selbstverständlich sind hier auch abweichende Formen denkbar, solange die in der Basisplatte 20 vorliegende Öffnung zur Aufnahme des Tauchrohrs 11 geeignet ist. Zur Fixierung des Tauchrohrs 11 an der Basisplatte 20 wird ein Haltering 21 auf den konusförmigen Kragen 11' aufgesetzt **(****Fig. 13****),** wobei der Haltering 21 eine korrespondierend zum Kragen 11' ausgebildete konusförmige Öffnung aufweist und mittels Bolzen 23 und Muttern 23b an dem Basisring 20 gehalten wird. Um auch hier etwa bei Temperatureinwirkung auftretende unterschiedliche Ausdehnungen aufzufangen, wird der Haltering 21 nicht starr fixiert, sondern es werden Federn 23a auf die Bolzen 23 zwischen den Haltering 21 und die Muttern 23b angeordnet, die zwar den Haltering 21 gegen den Kragen 11 pressen, allerdings bei Auftreten einer entsprechenden Gegenkraft auch eine weitere Kompression erlauben und damit zu große Spannungen vermeiden.

In dem offenen Ende des Tauchrohrs 11 in **Fig. 13** wird das Kopfteil 12 nach Einführen des Trägerkörpers 3 mit den LEDs 1 in das Tauchrohr 11 abdichtend angeordnet und fixiert (vgl. **Fig. 11****),** nachdem, falls erforderlich, das Kopfteil 12 mit dem Trägerkörper 3 elektrisch (Anschlussvorrichtungen 7', 7") und fluidisch (Anschlussleitung 18 und Abschnitt 18") verbunden wurde. Bevor abschließend die Abdeckplatte 22 ggf. unter Anordnung eines zylindrischen Gehäuses 26 mit der Basisplatte 20 über Bolzen 25 verbunden wird, die durch die Bohrungen 24 geführt werden, können die Anschlussleitungen 18, 18' mit einem entsprechenden Zu- und Ablauf für die nichtleitende Flüssigkeit und die elektrische Anschlussvorrichtung 7 mit einer entsprechenden elektrischen/elektronischen Leitung (nicht dargestellt) verbunden werden.

Die Erfindung ist nicht auf die dargestellten Beispiele beschränkt, die vorwiegend ein Lampenmodul mit einem einseitig geschlossenen Tauchrohr in vertikaler Anordnung zeigen. Selbstverständlich sind auch abweichende Anordnungen und Tauchrohre mit beidseitig offenem Ende denkbar, von denen ein Ende mit dem Kopfteil und das andere mit einem Verschlussteil verschlossen werden kann.

Ferner sind die in den Figuren beispielhaft gezeigten Ausführungsformen und Merkmalskombinationen nicht beschränkend gemeint. Abweichung in Anzahl und Anordnung der LEDs, Form und Dimensionierung von Trägerkörper, Kopfteil und Tauchrohr sind vom Schutzumfang ebenso umfasst wie abweichende Merkmalskombinationen hinsichtlich der Ausführung mit Wärmeleitstrukturen, Kühlmittelkreisläufen, und elektrischen Anschlussvorrichtungen etc.

Generell kann ein erfindungsgemäßes LED-Lampenmodul, wie in **Fig. 6** skizziert, einen oder mehrere Temperatursensoren auf der Trägerstruktur bzw. der Platine aufweisen, die für eine Schutzabschaltung zum Schutz der Halbleiterbauteile sorgt, wenn die maximal zulässige Umgebungstemperatur überschritten wird. Bei Unterschreiten der Maximaltemperatur kann die Steuerungsvorrichtung die entsprechenden LEDs wieder selbsttätig zuschalten. Auch bei Ausfall einer LED einer Platine können Sensoren dies erfassen und in der Steuerungsvorrichtung eine Abschaltung der Platine bzw. Trennung vom Stromkreis bei gleichzeitiger Anpassung der Gesamtstromstärke bewirken. Ein solcher LED-Ausfall wird von einem Temperatur-Hotspot begleitet, der bei einer Kettenreaktion und Ausfall mehrerer LEDs in kurzer Folge zu einer überhöhten Grenztemperatur führen kann, was daher zu vermeiden ist. Die weiteren Platinen können mit begrenzter Stromstärke weiter betrieben werden, sodass ein Totalausfall des Lampenmoduls verhindert wird. Sämtliche sicherheitsrelevante Sensoren des Lampenmoduls können redundant bzw. zweikanalig ausgeführt sein, um die entsprechend notwendige SIL Klasse zu realisieren.

In **Fig. 6** ist ferner eine Erkennungseinheit E skizziert, die zur Feststellung eines Ausfalls einer LED 1 vorgesehen ist. Die Erkennungseinheit E ist auf der Platine 30 angeordnet und dazu konfiguriert, einen Ausfall einer oder mehrerer LEDs 1 auf der Trägerplatine 30 festzustellen und an die Stromversorgungs- und Steuerungseinheit 16 zu übermitteln, die in Abhängigkeit eines festgestellten Ausfalls die Stromversorgung für die betroffene Platine 30 unterbricht und für die weiteren Trägerplatinen 30 begrenzt.

Da die LEDs gruppenweise auf den Trägerstrukturen untergebracht sind, können diese bei Ausfall einzeln ausgetauscht werden.

Ferner ist das erfindungsgemäße Lampenmodul geeignet, prozessspezifische Spektren bereitzustellen, wobei ferner mittels Regelkreis die Strahlungsintensität dem photochemischen Prozess angepasst werden kann. So kann eine Leistungsregelung der LEDs (Dimming) zur Prozesskontrolle erfolgen, da in vielen Reaktionen sich die Absorption während des Prozesses ändert. Hierauf kann durch gezielte Mess- und Regelkreise und Lampendimming reagiert werden, um ein effizientes System zu realisieren und Überbestrahlungen zu vermeiden.

Ein erfindungsgemäßes Lampenmodul kann monochromatische LEDs sowie eine Mischung von LEDs mit unterschiedlichen Emissionsspektren aufweisen, die die optimale Ausnutzung des Absorptionsspektrums der jeweiligen Reaktion bereitstellt. Gleiches gilt, wenn die Tauchlampen für Bioreaktoren eingesetzt werden sollen. Hier können LEDs mit unterschiedlichen Emissionswellenlängen auf einer Trägerstruktur implementiert werden, um optimale Wachstumsraten zu erzielen. In unterschiedlichen Wachstumsphasen bzw. unterschiedlichen Zellen können jeweils optimal gemischte Lichtspektren und -intensitäten zum optimierten Wachstum bereitgestellt werden.

Die nichtleitende Flüssigkeit, die in dem erfindungsgemäßen Lampenmodul eingesetzt wird, wird dann in Abhängigkeit des Emissionsspektrums der LED ausgewählt, damit die nichtleitende Flüssigkeit eine ausreichende Transmission aufweist, um die verbesserte Gesamtlichtleistung des Lampenmoduls bereitzustellen.

### BEZUGSZEICHENLISTE

- 1: LED, OLED
- 1': Primäroptiklose LED
- 1a: Primäroptik/Kunststofflinse
- 1b: Halbleiterkristall
- 1c: Draht
- 1d: Anode
- 1e: LED-Chip/Phosphorschicht
- 1f: Kathode
- 1g: Träger
- 2: Wärmeleitstruktur
- 3: Trägerkörper
- 3', 3": Innenstruktur, Öffnung
- 3a, 3b: Sockelabschnitt
- 3c: Ringfeder
- 4: Kühlzufuhrabschnitt
- 5: längswasserdichter Anschluss
- 6: Kammer
- 7, 7': elektrische Anschlussvorrichtung
- 7": Verbindungselement

- 9: mechanische Anschlussvorrichtung
- 10: Lampenmodul
- 11: Tauchrohr
- 11', 11": Konischer Kragen, Ringnut
- 12: Kopfteil
- 12': Flanschabschnitt
- 120: Schwenkriegel
- 120': Flügelabschnitt

- 14: Halterung
- 15: Stromversorgungs- und/oder Steuerungsleitung
- 15': Kontaktelement
- 16: Stromversorgungs- und Steuerungsvorrichtung
- 17: Dichtung
- 18,18': Kreislaufanschlussleitung
- 18": Anschlussabschnitt
- 19: Tauchrohrinnenraum
- 20: Basisring
- 21: Haltering
- 21': Anpressring
- 22: Abdeckplatte
- 23: Stehbolzen
- 23a: Feder
- 23b: Mutter
- 24: Bohrung
- 25: Bolzen
- 26: Gehäuse
- 30: Trägerplatine
- 100: nichtleitende Flüssigkeit

- A: Armatur
- AT: Atmungseinheit mit Trocknungsmittel
- E: Erkennungseinheit
- F: Durchflussmesser
- P: Pumpe
- S: Steuerungseinheit
- T: Temperatursensor
- WT: Wärmetauscher

## Patentansprüche

1. Lampenmodul (10), das dazu ausgebildet ist, als Tauchstrahler in photochemischen Reaktoren eingesetzt zu werden, das
einen Trägerkörper (3) mit zumindest einer Licht emittierenden Diode (LED) (1) und ein Kopfteil (12) zum elektrischen Anschluss der zumindest einen LED (1) und zur Halterung des Trägerkörpers (3) und
ein Tauchrohr (11) aufweist, das einen Tauchrohrinnenraum (19) begrenzt, in dem der Trägerkörper (3) mit der zumindest einen LED (1) angeordnet ist,
wobei das Kopfteil (12) Anschlussleitungen (18, 18') für die Zufuhr und die Ableitung einer Kühlflüssigkeit aufweist, und der Trägerkörper (3) als Kühlkörper ausgebildet ist, der zumindest einen internen Fluidpfad als Zufuhrabschnitt (4) für die Kühlflüssigkeit begrenzt, wobei der Zufuhrabschnitt (4) über das Kopfteil (12) mit einer ersten Anschlussleitung (18) verbunden ist,
**dadurch gekennzeichnet, dass**
die Kühlflüssigkeit eine elektrisch nichtleitende Flüssigkeit (100) ist, die für die Wellenlängen der von den LEDs (1) des Lampenmoduls (10) emittierten Strahlung transparent ist, und
der von dem Tauchrohr (11) begrenzte Tauchrohrinnenraum (19) derart mit der elektrisch nichtleitenden Flüssigkeit (100) gefüllt ist, dass die zumindest eine LED (1) vollständig in die nichtleitende Flüssigkeit (100) eingetaucht ist,
wobei die Anschlussleitungen (18, 18') für die Zufuhr und die Ableitung der nichtleitenden Flüssigkeit (100) mit dem Tauchrohrinnenraum (19) kommunizieren, wobei der Zufuhrabschnitt (4), der über das Kopfteil (12) mit der ersten Anschlussleitung (18) verbunden ist, an einer von dem Kopfteil (12) abgewandten Seite des Trägerkörpers (3) in den Tauchrohrinnenraum (19) mündet, und wobei sich eine zweite Anschlussleitung (18'), die sich durch das Kopfteil (12) erstreckt, am Kopfteil (12) zum Tauchrohrinnenraum (19) öffnet,
wobei
die Anschlussleitungen (18, 18') zur Bildung eines Kreislaufs der nichtleitenden Flüssigkeit (100) verbunden sind, wobei der Kreislauf zumindest einen Wärmetauscher (WT) und eine Fördervorrichtung (P) aufweist.

2. Lampenmodul (10) nach Anspruch 1,
**dadurch gekennzeichnet, dass**
das Kopfteil (12) mittels zumindest einer Dichtung (17) abdichtend mit einem offenen Ende des Tauchrohrs (11) verbunden ist, wobei die abdichtende Verbindung bevorzugt federgelagert und/oder formschlüssig ist.

3. Lampenmodul (10) nach zumindest einem der Ansprüche 1 oder 2,
**dadurch gekennzeichnet, dass**
das Tauchrohr (11) mit dem Kopfteil (12) an dem offenen Ende einen sich vom offenen Ende aus aufweitenden, konusförmigen Kragen (11') aufweist, der von einem Haltering (21) mit einer korrespondierend konusförmigen Öffnung an einem Basisring (20) mittels federbelasteter Bolzen (23) gehalten wird, wobei das Kopfteil (12) in dem offenen Ende des Tauchrohrs (11) aufgenommen und durch Schwenkriegel (120), die mit einer Ringnut (11") an einer Innenwand des Tauchrohrs (11) in Eingriff bringbar sind, oder bevorzugt durch einen Anpressring (21'), dessen Öffnung einen Durchmesser hat, der kleiner ist als der Durchmesser des Kopfteils (12) und der auf dem Haltering (21) angeordnet und zusammen mit dem Haltering (21) an einem Basisring (20) mittels federbelasteter Bolzen (23) gehalten wird, abdichtend befestigt wird.

4. Lampenmodul (10) nach einem der Ansprüche 2 oder 3,
**dadurch gekennzeichnet, dass**
das Lampenmodul (10) vorzugsweise zusätzlich einen Durchflussmesser (F) aufweist, der besonders bevorzugt ein Massendurchflussmesser, beispielsweise ein Coriolis-Massendurchflussmesser oder Schwebekörperdurchflussmesser ist, wobei der Durchflussmesser (F) mit einer Steuerungseinheit (S) verbunden ist, die dazu konfiguriert ist, die Fördervorrichtung (P) und/oder eine in einer der Anschlussleitungen (18, 18') vorgesehene Armatur (A) in Abhängigkeit des von dem Durchflussmesser (F) gemessenen Durchflusswerts zu steuern, um eine vorbestimmte Fließgeschwindigkeit der nichtleitenden Flüssigkeit (100) durch den Tauchrohrinnenraum (19) entlang einer Oberfläche der LEDs (1) einzuhalten.

5. Lampenmodul (10) nach zumindest einem der Ansprüche 1 bis 4,
**dadurch gekennzeichnet, dass**
die nichtleitende Flüssigkeit (100) aus hochraffinierten Mineralölen, Silikonölen und synthetischen Ester- oder Etherverbindungen ausgewählt ist, wobei die nichtleitende Flüssigkeit (100) eine Viskosität bei 25ºC von 5 bis 60 cSt, vorzugsweise von 20 bis 50 cSt, aufweist.

6. Lampenmodul (10) nach zumindest einem der Ansprüche 1 bis 5,
**dadurch gekennzeichnet, dass** das Kopfteil (12) und/oder der Trägerkörper (3) Wärmeableitstrukturen (2) aufweist.

7. Lampenmodul (10) nach zumindest einem der Ansprüche 1 bis 6,
**dadurch gekennzeichnet, dass**
das Tauchrohr (11) ein doppelwandiges Tauchrohr (11) ist oder das Lampenmodul (10) ein zweites Tauchrohr aufweist, in dem das erste Tauchrohr (11) angeordnet ist.

8. Lampenmodul (10) nach zumindest einem der Ansprüche 1 bis 7,
**dadurch gekennzeichnet, dass**
der Trägerkörper (3) zumindest eine Kammer (6) aufweist, durch die sich zumindest eine Stromversorgungs- und/oder Steuerungsleitung (15) von dem Kopfende des Trägerkörpers (3) bis zu einem Kontaktelement (15') der zumindest einen LED (1) erstreckt.

9. Lampenmodul (10) nach Anspruch 8,
**dadurch gekennzeichnet, dass**
das Kopfteil (12) zumindest eine elektrische Anschlussvorrichtung (7) aufweist, wobei bevorzugt das Kopfteil (12) eine Stromversorgungs- und Steuerungsvorrichtung (16) für die zumindest eine LED (1) des Lampenmoduls (10) enthält und die zumindest eine Stromversorgungs- und/oder Steuerungsleitung (15) über die Stromversorgungs- und Steuerungsvorrichtung (16) mit der zumindest einen elektrischen Anschlussvorrichtung (7) verbunden ist,
und wobei bevorzugt die sich durch das Kopfteil (12) erstreckenden Anschlussleitungen (18, 18') zur Kühlung der Stromversorgungs- und Steuerungsvorrichtung (16) ausgebildet sind,
und/oder an dem Kopfteil (12) zumindest eine mechanische Anschlussvorrichtung (9) zur Verbindung des Kopfteils (12) mit einer Halterung (14) vorliegt.

10. Lampenmodul (10) nach zumindest einem der Ansprüche 1 bis 9,
**dadurch gekennzeichnet, dass**
das Lampenmodul (10) eine Mehrzahl von LEDs (1) aufweist, wobei jeweils eine Teilanzahl der Mehrzahl der LEDs (1) auf jeweils einer Trägerplatine (30) angeordnet ist und die Trägerplatinen (30) an dem Trägerkörper (3) befestigt sind.

11. Lampenmodul (10) nach Anspruch 9 oder 10,
**dadurch gekennzeichnet, dass**
- das Lampenmodul (10) zumindest einen Temperatursensor aufweist, der auf dem Trägerkörper (3) oder der Trägerplatine (30) angeordnet und mit der Stromversorgungs- und Steuerungsvorrichtung (16) des Lampenmoduls (10), die einen Schutzschalter für die LEDs (1) umfasst, verbunden ist, und/oder dass
- das Lampenmodul (10) eine Erkennungseinheit (E) zur Feststellung eines Ausfalls zumindest einer LED (1) aufweist, die mit der Stromversorgungs- und Steuerungsvorrichtung (16) des Lampenmoduls (10) verbunden und dazu konfiguriert ist, einen Ausfall einer oder mehrerer LEDs (1) auf dem Trägerkörper (3) oder einer der Trägerplatinen (30) festzustellen, wobei die Erkennungseinheit (E) oder Stromversorgungs- und Steuerungsvorrichtung (16) dazu ausgebildet ist, in Abhängigkeit eines festgestellten Ausfalls die Stromversorgung für die weiteren LEDs (1) oder die weiteren Trägerplatinen (30) zu begrenzen oder zu unterbrechen, und/oder dass
- die Stromversorgungs- und Steuerungsvorrichtung (16) zumindest einen Regelkreis für eine LED-Ansteuerung aufweist, mit dem gleichartige oder unterschiedliche LEDs (1) dimmbar sind und/oder das Spektrum der emittierten Wellenlängen unterschiedlicher LEDs (1) änderbar ist.

12. Lampenmodul (10) nach zumindest einem der Ansprüche 1 bis 11,
**dadurch gekennzeichnet, dass**
das Lampenmodul (10) in einer der Anschlussleitungen (18, 18') eine Atmungseinheit mit Trocknungsmittel (AT) aufweist.

13. Lampenmodul (10) nach zumindest einem der Ansprüche 1 bis 12,
**dadurch gekennzeichnet, dass**
zumindest eine LED (1) des Lampenmoduls (10), bevorzugt alle LEDs (1) des Lampenmoduls (10) ohne Primäroptik (1a) ausgeführt ist.

14. Photoreaktor mit einer darin angeordneten Lampe mit einem für die photochemische Reaktion geeigneten Emissionsspektrum,
**dadurch gekennzeichnet, dass**
die Lampe ein Lampenmodul (10) nach zumindest einem der Ansprüche 1 bis 13 ist.

## Claims

1. Lamp module (10) that is configured to be used as an immersion radiator in photochemical reactors, that comprises
a support body (3) with at least one light-emitting diode (LED) (1) and a head part (12) for electrical connection of the at least one LED (1) and for holding the support body (3) and
an immersion pipe (11) that delimits an immersion pipe interior (19) in which the support body (3) with the at least one LED (1) is arranged, wherein the head part (12) comprises connection lines (18, 18') for the supply and the discharge of a cooling liquid, and the support body (3) is configured as a cooling body that delimits at least one internal fluid path as supply section (4) for the cooling liquid, wherein the supply section (4) is connected through the head part (12) to a first connection line (18),
**characterized in that**
the cooling liquid is an electrically non-conductive liquid (100) that is transparent for the wavelengths of the radiation emitted by the LEDs (1) of the lamp module (10), and
the immersion pipe interior (19) delimited by the immersion pipe (11) is filled with the electrically non-conductive liquid (100) such that the at least one LED (1) is immersed completely in the non-conductive liquid (100),
wherein the connection lines (18, 18') for the supply and the discharge of the non-conductive liquid (100) communicate with the immersion pipe interior (19), wherein the supply section (4) that is connected through the head part (12) to the first connection line (18) opens at a side of the support body (3) facing away from the head part (12) into the immersion pipe interior (19), and wherein a second connection line (18') that extends through the head part (12) opens at the head part (12) toward the immersion pipe interior (19),
wherein the connection lines (18, 18') are connected for forming a circuit of the non-conductive liquid (100), wherein the circuit comprises at least one heat exchanger (WT) and a conveying device (P).

2. Lamp module (10) according to claim 1,
**characterized in that**
the head part (12) is connected by means of at least one seal (17) sealingly to an open end of the immersion pipe (11), wherein the sealing connection is preferably spring-supported and/or a form fit connection.

3. Lamp module (10) according to at least one of the claims 1 or 2,
**characterized in that**
the immersion pipe (11) with the head part (12) at the open end comprises a cone-shaped collar (11') widening away from the open end, which is held by a holding ring (21) with a corresponding cone-shaped opening at a base ring (20) by means of spring-loaded bolts (23), wherein the head part (12) is received in the open end of the immersion pipe (11) and is fastened seal-tightly by pivot latches (120) that can be brought into engagement with an annular groove (11") at an inner wall of the immersion pipe (11), or preferably by a pressure ring (21') whose opening has a diameter that is smaller than the diameter of the head part (12) and which is arranged on the holding ring (21) and together with the holding ring (21) is held at a base ring (20) by means of spring-loaded bolts (23).

4. Lamp module (10) according to one of the claims 2 or 3,
**characterized in that**
the lamp module (10) preferably comprises additionally a flow meter (F) that is particularly preferred a mass flow meter, for example, a Coriolis mass flow meter or floating body flow meter, wherein the flow meter (F) is connected to a control unit (S) that is configured to control the conveying device (P) and/or a fitting (A) provided at one of the connection lines (18, 18') depending on the flow values measured by the flow meter (F) in order to maintain a predetermined flow rate of the non-conductive liquid (100) through the immersion pipe interior (19) along a surface of the LEDs (1).

5. Lamp module (10) according to at least one of the claims 1 to 4,
**characterized in that**
the non-conductive liquid (100) is selected from highly refined mineral oils, silicone oils, and synthetic ester or ether compounds, wherein the non-conductive liquid (100) comprises a viscosity at 25°C of 5 to 60 cSt, preferably of 20 to 50 cSt.

6. Lamp module (10) according to at least one of the claims 1 to 5,
**characterized in that**
the head part (12) and/or the support body (3) comprises heat dissipating structures (2).

7. Lamp module (10) according to at least one of the claims 1 to 6,
**characterized in that**
the immersion pipe (11) is a double-wall immersion pipe (11) or the lamp module (10) comprises a second immersion pipe in which the first immersion pipe (11) is arranged.

8. Lamp module (10) according to at least one of the claims 1 to 7,
**characterized in that**
the support body (3) comprises at least one chamber (6) through which at least one current supply and/or control line (15) extends from the head end of the support body (3) to a contact element (15') of the at least one LED (1).

9. Lamp module (10) according to claim 8,
**characterized in that**
the head part (12) comprises at least one electrical connection device (7) wherein preferably the head part (12) contains a current supply and control device (16) for the at least one LED (1) of the lamp module (10) and the at least one current supply and/or control line (15) is connected via the current supply and control device (16) to the at least one electrical connection device (7),
and wherein preferably the connection lines (18, 18') extending through the head part (12) are configured for cooling the current supply and control device (16),
and/or at least one mechanical connection device (9) for connecting the head part (12) to a holder (14) is present at the head part (12).

10. Lamp module (10) according to at least one of the claims 1 to 9,
**characterized in that**
the lamp module (10) comprises a multitude of LEDs (1) wherein, respectively, a portion of the multitude of the LEDs (1) are arranged on a support circuit board (30), respectively, and the support circuit boards (30) are fastened to the support body (3).

11. Lamp module (10) according to claim 9 or 10,
**characterized in that**
- the lamp module (10) comprises at least one temperature sensor that is arranged on the support body (3) or the support circuit board (30) and is connected to the current supply and control device (16) of the lamp module (10) that comprises a protective switch for the LEDs (1), and/or **in that**
- the lamp module (10) comprises a detection unit (E) for determining a failure of at least one LED (1) that is connected to the current supply and control device (16) of the lamp module (10) and is configured to determine a failure of one or a plurality of the LEDs (1) on the support body (3) or one of the support circuit boards (30), wherein the detection unit (E) or current supply and control device (16) is configured to delimit or to interrupt, depending on a determined failure, the current supply for the further LEDs (1) or the further support circuit boards (30), and/or **in that**
- the current supply and control device (16) comprises at least one control circuit for an LED control with which same type or different LEDs (1) can be dimmed and/or the spectrum of the emitted wavelengths of different LEDs (1) can be changed.

12. Lamp module (10) according to at least one of the claims 1 to 11,
**characterized in that**
the lamp module (10) comprises a breathing unit with drying agent (AT) in one of the connection lines (18, 18').

13. Lamp module (10) according to at least one of the claims 1 to 12,
**characterized in that**
at least one LED (1) of the lamp module (10), preferably all LEDs (1) of the lamp module (10), are configured without primary optic (1a).

14. Photoreactor with a lamp arranged therein with an emission spectrum suitable for the photochemical reaction,
**characterized in that**
the lamp is a lamp module (10) according to at least one of the claims 1 to 13.

## Revendications

1. Module de lampe (10) conçu pour être utilisé comme lampe à immersion dans des réacteurs photochimiques, comprenant
un corps porteur (3) avec au moins une diode électroluminescente (LED) (1) et une partie supérieure (12) pour le raccordement électrique de la au moins une LED (1) et pour le maintien du corps porteur (3) et
un tube plongeur (11) qui délimite un espace intérieur du tube plongeur (19) dans lequel est disposé le corps porteur (3) avec la au moins une LED (1), la partie supérieure (12) présentant des lignes de raccordement (18, 18') pour l'amenée et l'évacuation d'un liquide de refroidissement, et le corps porteur (3) étant conçu comme un corps de refroidissement qui limite au moins un chemin de fluide interne comme section d'amenée (4) pour le liquide de refroidissement, la section d'amenée (4) étant reliée à une première ligne de raccordement (18) par la partie supérieure (12),
**caractérisé en ce que**
le liquide de refroidissement est un liquide électriquement non conducteur (100) qui est transparent aux longueurs d'onde du rayonnement émis par les LED (1) du module de lampe (10), et
l'espace intérieur du tube plongeur (19), délimité par le tube plongeur (11), est rempli du liquide électriquement non conducteur (100) de telle manière à ce que la au moins une LED (1) soit complètement immergée dans le liquide non conducteur (100),
les lignes de raccordement (18, 18') pour l'amenée et l'évacuation du liquide non conducteur (100) communiquant avec l'espace intérieur du tube plongeur (19), la section d'amenée (4) qui est reliée à la première ligne de raccordement (18) par la partie supérieure (12), s'ouvrant sur un côté du corps porteur (3), écarté de la partie supérieure (12), dans l'espace intérieur du tube plongeur (19), et une deuxième ligne de raccordement (18') qui s'étend par la partie supérieure (12), s'ouvrant sur la partie supérieure (12) vers l'espace intérieur du tube plongeur (19),
les lignes de raccordement (18, 18') pour la formation d'un circuit du liquide non conducteur (100) étant reliées, le circuit présentant au moins un échangeur de chaleur (WT) et un convoyeur (P).

2. Module de lampe (10) selon la revendication 1,
**caractérisé en ce que**
la partie supérieure (12) est reliée de manière étanche à une extrémité ouverte du tube plongeur (11) au moyen au moins d'un joint d'étanchéité (17), la liaison d'étanchéité étant de préférence montée sur ressort et/ou mécaniquement.

3. Module de lampe (10) selon au moins l'une des revendications 1 ou 2, **caractérisé en ce que**
le tube plongeur (11) avec la partie supérieure (12) présente, à l'extrémité ouverte, une collerette conique (11'), s'élargissant depuis l'extrémité ouverte, qui est maintenue par un anneau de retenue (21) présentant une ouverture conique correspondante sur un anneau de base (20) au moyen de boulons sur ressort (23), la partie supérieure (12) étant logée dans l'extrémité ouverte du tube plongeur (11) et étant fixée de manière étanche par des verrous pivotants (120) qui peuvent être mis en prise sur une paroi intérieure du tube plongeur (11) avec une rainure annulaire (11"), ou de préférence par un anneau de pression (21'), dont l'ouverture a un diamètre inférieur au diamètre de la partie supérieure (12), et qui est disposé sur l'anneau de retenue (21) et maintenu avec l'anneau de retenue (21) sur un anneau de base (20) au moyen de boulons sur ressort (23).

4. Module de lampe (10) selon l'une des revendications 2 ou 3,
**caractérisé en ce que**
le module de lampe (10) présente préférablement en outre un débitmètre (F) qui plus préférablement un débitmètre massique, par exemple un débitmètre massique de Coriolis ou un débitmètre à flotteur, le débitmètre (F) étant relié à une unité de commande (S) qui est configurée pour commander le convoyeur (P) et/ou un robinet (A) prévu dans une des lignes de raccordement (18, 18') en fonction de la valeur de débit mesurée par le débitmètre (F), afin de maintenir une vitesse d'écoulement prédéterminée du liquide non conducteur (100) à travers l'espace intérieur du tube plongeur (19) le long d'une surface des LED (1).

5. Module de lampe (10) selon au moins l'une des revendications 1 à 4, **caractérisé en ce que**
le liquide non conducteur (100) est choisi parmi des huiles minérales hautement raffinées, des huiles de silicone et des composés d'ester ou d'éther synthétiques, le liquide non conducteur (100) ayant une viscosité de 5 à 60 cSt à 25°C, préférablement de 20 à 50 cSt.

6. Module de lampe (10) selon au moins l'une des revendications 1 à 5, **caractérisé en ce que**
la partie supérieure (12) et/ou le corps porteur (3) présente des structures de dissipation de la chaleur (2).

7. Module de lampe (10) selon au moins l'une des revendications 1 à 6, **caractérisé en ce que**
le tube plongeur (11) est un tube plongeur (11) à double paroi ou le module de lampe (10) présente un deuxième tube plongeur dans lequel est disposé le premier tube plongeur (11).

8. Module de lampe (10) selon au moins l'une des revendications 1 à 7, **caractérisé en ce que**
le corps porteur (3) comprend au moins une chambre (6) à travers laquelle s'étend au moins une ligne d'alimentation électrique et/ou de commande (15) depuis l'extrémité supérieure du corps porteur (3) jusqu'à un élément de contact (15') de la au moins une LED (1).

9. Module de lampe (10) selon la revendication 8,
**caractérisé en ce que**
la partie supérieure (12) présente au moins un dispositif de raccordement électrique (7), la partie supérieure (12) comprenant préférablement un dispositif d'alimentation électrique et de commande (16) pour la au moins une LED (1) du module de lampe (10) et la au moins une ligne d'alimentation électrique et/ou de commande (15) étant reliée au moins au un dispositif de raccordement électrique (7) par le dispositif d'alimentation électrique et de commande (16),
et les lignes de raccordement (18, 18') s'étendant à travers la partie supérieure (12) étant préférablement conçues pour le refroidissement du dispositif d'alimentation électrique et de commande (16),
et/ou au moins un dispositif de raccordement mécanique (9) pour relier la partie supérieure (12) à un support (14) étant disponible sur la partie supérieure (12).

10. Module de lampe (10) selon au moins l'une des revendications 1 à 9, **caractérisé en ce que**
le module de lampe (10) présente une pluralité de LED, un nombre partiel de la pluralité des LED (1) étant à chaque fois disposé sur une platine support (30) et les platines support (30) étant fixées au corps porteur (3).

11. Module de lampe (10) selon la revendication 9 ou 10,
**caractérisé en ce que**
- le module de lampe (10) présente au moins une sonde de températurequi est disposée sur le corps porteur (3) ou la platine support (30) et qui est reliée au dispositif d'alimentation électrique et de commande (16) du module de lampe (10) qui comprend un disjoncteur pour les LED (1), et/ou **en ce que**
- le module de lampe (10) présente une unité de détection (E) pour détecter une défaillance au moins d'une LED (1), laquelle est reliée au dispositif d'alimentation électrique et de commande (16) du module de lampe (10) et configurée pour détecter une défaillance d'une ou plusieurs LED (1) sur le corps porteur (3) ou l'une des platines support (30), l'unité de détection (E) ou le dispositif d'alimentation électrique et de commande (16) étant conçu pour limiter ou interrompre l'alimentation électrique pour les autres LED (1) ou les autres platines support (30) en fonction d'une défaillance détectée, et/ou **en ce que**
- le dispositif d'alimentation électrique et de commande (16) présente au moins un circuit de régulation pour une commande des LED, permettant de faire varier l'intensité des LED (1) de même type ou de types différents et/ou de modifier le spectre des longueurs d'onde émises par les différentes LED (1).

12. Module de lampe (10) selon au moins l'une des revendications 1 à 11, **caractérisé en ce que**
le module de lampe (10) dans une des lignes de raccordement (18, 18') présente une unité de ventilation avec un agent desséchant (AT).

13. Module de lampe (10) selon au moins l'une des revendications 1 à 12, **caractérisé en ce que**
au moins une LED (1) du module de lampe (10), préférablement toutes les LED (1) du module de lampe (10), est conçue sans optique primaire (1a).

14. Photoréacteur avec une lampe disposée à l'intérieur, avec un spectre d'émission approprié à la réaction photochimique,
**caractérisé en ce que**
la lampe est un module de lampe (10) conformément au moins à l'une des revendications 1 à 13.
